# EUROPÄISCHE PATENTSCHRIFT

(11) **EP 1 569 612 B1**
(45) Veröffentlichungstag und Bekanntmachung des Hinweises auf die Patenterteilung: **09.07.2008**
(21) Anmeldenummer: 03812182.8
(22) Anmeldetag: 21.11.2003
(51) Int. Cl.: A61K 8/49, A61K 31/429, A61Q 17/04, A61Q 19/00, A61P 17/06, A61P 17/10

(54) **NEUE TOPISCHE VERWENDUNG VON BIS-ARYLIMIDAZO 1,2-A THIOLANDERIVATEN**
NOVEL TOPICAL USE OF BIS-ARYLIMIDAZO 1,2-A THIOLANE DERIVATIVES
NOUVELLE UTILISATION TOPIQUE DE DERIVES DE BIS-ARYLIMIDAZO 1,2-A THIOLANE

(30) Priorität: 05.12.2002 DE 10256881
(43) Veröffentlichungstag der Anmeldung: 07.09.2005
(73) Patentinhaber: Beiersdorf AG, 20245 Hamburg (DE)
(72) Erfinder: KOLBE, Ludger, 21255 Dohren (DE); PFANNENBECKER, Uwe, 22419 Hamburg (DE); KRUSE, Inge, 20146 Hamburg (DE); SOKOLOWSKI, Tobias, 20257 Hamburg (DE); IMMEYER, Jeannine, 21272 Egestorf-Sahrendorf (DE); TOM DIECK, Karen, Hamburg 22299 (DE); DANNHARDT, Gerd, 55127 Mainz (DE)
(86) Internationale Anmeldenummer: PCT/EP2003/050865
(87) Internationale Veröffentlichungsnummer: WO 2004/050051

(56) Entgegenhaltungen:
- EP-A- 0 231 622
- US-A- 4 064 260
- BENDER P E ET AL: "5 6 DIARYL-2 3-DIHYDROIMIDAZO-2 1-B-THIAZOLES A NEW CLASS OF IMMUNOREGULATORY ANTIINFLAMMATORY AGENTS" JOURNAL OF MEDICINAL CHEMISTRY, Bd. 28, Nr. 9, 1985, Seiten 1169-1177, XP002272601 & ISSN: 0022-2623

## Beschreibung

Die vorliegende Erfindung betrifft neuartige Verwendungen von bestimmten Bis-Arylimidazo[1,2-a]thiolanderivaten. Bis-Arylimidazole wurden in der Vergangenheit breit untersucht, um antientzündlich wirksame Stoffe zu finden. In vielen Fällen gelang dies auch. Ein großer Teil dieser Anstrengungen richtete sich allerdings auf Arzneipräparate für die orale Einnahme, aber nicht zum Auftragen auf die Haut. Dies hat zumindest zum Teil seine Ursache darin, daß viele Cyclooxygenase-Inhibitoren, zu denen auch die hier beschriebenen Bis-Arylimidazole gehören, für ihre photocytotoxischen Eigenschaften bekannt sind (Acta Derm Venereol 1996,76:337-40).

Antientzündlich wirksame Zubereitungen spielen in der Kosmetik und Dermatologie eine große Rolle. Solche Zubereitungen haben unterschiedliche Anwendungsgebiete, zum Beispiel die Linderung des Rasurbrandes.

Der Wuchs des Barthaares wird beim heranwachsenden Mann durch die gesteigerte Bildung männlicher Hormone während der Pubertät ausgelöst. Hormonelle Störungen bei der Frau können ebenfalls zu einer Form von Bartwuchs führen, die allerdings in aller Regel in seiner Ausprägung deutlich hinter dem des männlichen Bartwuchses zurückbleibt.

Die Rasur des Gesichts oder anderer behaarter Körperteile (wie beispielsweise der Beine, der Achsel oder des Intimbereichs) kann durch mancherlei Zwänge - z. B. religiöser oder kultureller Art - motiviert werden; im einfachsten Fall ist der Haarwuchs für die betreffende Person schlichtweg aus kosmetischen Gründen unerwünscht.

Eine Rasur wird entweder trocken oder naß durchgeführt. Die Entwicklung von neuen mechanischen und elektrischen Naß- und Trockenrasiertechniken ermöglicht heutzutage ein sicheres und gründliches Entfernen des (Bart-) Haares. Bei der Naßrasur sind in der Regel chemische Hilfsmittel - beispielsweise in Form von Rasiergelen, -seifen oder -schäumen - unerläßlich.

Diese werden benötigt, um das (Bart-) Haar aufweichen und so den zum Durchschneiden erforderlichen Kraftaufwand - und damit das unangenehme Ziehen am Haarschaft-zu minimieren. Das Aufweichen der (Bart-) Haare wird durch Wasseraufnahme erreicht, die durch die Erhöhung des pH-Werts der Haare ermöglicht wird. Naßrasurmittel enthalten deshalb in der Regel Seifen bzw. Fettsäuresalze, deren pH-Wert im Bereich von 8-10 liegt. Produkte für die Naßrasur erzeugen daher ein typisches Hautgefühl, welches nach der Anwendung auftritt. Die Haut fühlt sich trocken und rauh an. Dieses Hautgefühl wird in der kosmetischen Fachwelt auch als "squeaky-feeling" bezeichnet und ist bei Verbrauchern und Verbraucherinnen äußerst unbeliebt.

Auch bei der Trockenrasur sind kosmetische Mittel häufig empfehlenswert, um eine möglichst dichte Rasur zu bewirken, d. h. das (Bart-) Haar möglichst nahe an der Hautoberfläche abzuschneiden.

Die von der Rasur betroffenen Hautpartien können allerdings nicht nur durch die Rasurhilfsmittel gereizt werden, auch die mechanische Reizung durch das Rasieren an sich stellt eine Belastung der Haut dar, welche ein unangenehmes Hautgefühl (den sog. "Rasurbrand") zur Folge haben kann.

Eine Aufgabe der vorliegenden Erfindung war es daher, kosmetische oder dermatologische Zubereitungen zu finden, die die Nachreaktionen der Haut auf die (mechanische) Reizung durch das Rasieren besser vermindern.

Darüberhinaus betrifft die Erfindung Zubereitungen mit extrem niedrigem sogenanntem "Stinging Potential".

Das Problem "sensible Haut" betrifft eine wachsende Anzahl Erwachsenen und Kindern. Mit Sensibler Haut bezeichnet man eine Kombination verschiedener Symptome, wie hyperreaktive und intolerante Haut. Aber auch atopische Haut kann darunter subsummiert werden. Diese Hautzustände werden von den Betroffenen oftmals, nicht ganz korrekt, als allergische" Haut bezeichnet. Obwohl eine allergische Erkrankung zu Symptomen der sensiblen Haut führen kann, ist die Erscheinung "sensible Haut" nicht auf Allergiker beschränkt.

Die Haut, insbesondere die Epidermis, ist als Barriereorgan des menschlichen Organismus in besonderem Maße äußeren Einwirkungen unterworfen. Nach dem heutigen wissenschaftlichen Verständnis repräsentiert die Haut ein immunologisches Organ, das als immunkompetentes peripheres Kompartiment eine eigene Rolle in induktiven, effektiven und regulativen lmmunprozessen des Gesamtorganismen spielt.

Die Epidermis ist reich mit Nerven und Nervenendapparaten wie Vater-Pacini-Lamellenkörpern, Merkel-Zell-Neuritenkomplexen und freien Nervenendigungen für Schmerz-, Kälte-, Wärmeempfindung und Juckreiz ausgestattet.

Bei Menschen mit sensibler, empfindlicher oder verletzlicher Haut kann ein mit "Stinging" (<engl.> "to sting" = verletzen, brennen, schmerzen) bezeichnetes neurosensorisches Phänomen beobachtet werden. Diese "sensible Haut" unterscheidet sich grundsätzlich von "trockener Haut" mit verdickten und verhärteten Homschichten.

Typische Reaktionen des "Stinging" bei sensibler Haut sind Rötung, Spannen und Brennen der Haut sowie Juckreiz.

Als neurosensorisches Phänomen ist der Juckreiz bei atopischer Haut anzusehen, sowie Juckreiz bei Hauterkrankungen.

"Stinging"-Phänomene können als kosmetisch zu behandelnde Störungen angesehen werden. Starker Juckreiz dagegen, insbesondere bei Atopie auftretendes starkes Hautjucken, kann auch als schwerwiegendere dermatologische Störung bezeichnet werden.

Typische, mit den Begriffen "Stinging" oder "empfindlicher Haut" in Verbindung gebrachte, störende neurosensorische Phänomene sind Hautrötung, Kribbeln, Prickeln, Spannen und Brennen der Haut und Juckreiz. Sie können durch stimulierende Umgebungsbedingungen z.B. Massage, Tensideinwirkung, Wettereinfluß wie Sonne, Kälte, Trockenheit, aber auch feuchte Wärme, Wärmestrahlung und UV-Strahlung, z.B. der Sonne, hervorgerufen werden.

In "Journal of the Society of Cosmetic Chemists" 28, S.197 - 209 (Mai 1977) beschreiben P.J.Frosch und A.M.Kligman eine Methode zur Abschätzung des "Stinging-Potentials" topisch verabreichter Substanzen. Als positive Substanzen werden hier z.B. Milchsäure und Brenztraubensäure eingesetzt. Bei Messung nach dieser Methode wurden aber auch Aminosäuren, insbesondere Glycin, als neurosensorisch aktiv ermittelt (solche Substanzen werden "Stinger" genannt).

Nach bisherigen Erkenntnissen tritt eine derartige Empfindlichkeit gegenüber ganz bestimmten Substanzen individuell unterschiedlich auf. Dies bedeutet, eine Person, die bei Kontakt mit einer Substanz "Stingingeffekte" erlebt, wird sie mit hoher Wahrscheinlichkeit bei jedem weiteren Kontakt wiederholt erleben. Der Kontakt mit anderen "Stingern" kann aber ebensogut ohne jede Reaktion verlaufen.

Viele mehr oder weniger empfindliche Personen haben auch bei Verwendung mancher desodorierenden oder antitranspirierend wirkenden Zubereitungen unter erythematösen Hauterscheinungen zu leiden.

Erythematöse Hauterscheinungen treten auch als Begleiterscheinungen bei gewissen Hauterkrankungen oder -unregelmäßigkeiten auf. Beispielsweise ist der typische Hautausschlag beim Erscheinungsbild der Akne regelmäßig mehr oder weniger stark gerötet.

Das Blatt Chemical Abstract, Band 111 Nr. 97243c vom 11.9.1989 offenbart 2-(4,5-Diphenylimidazol-(2-pyridylmethyl)-sulfid, aber nicht die Methoxy- oder Chlor-Arylderivate.

Die Schrift JP 1040467 A offenbart Heterocyclen-alkylen-thiosubstituiere Phenylimidazole, jedoch keine 4,5-Bis(p-Methoxyphenyl)imidazolderivate.

Das Blatt Chemical Abstract, Band 71 Nr. 112863f vom 8.12.1969 offenbart 2-(4,5-Diphenylimidazol-(2-pyridylethyl)-sulfid, jedoch keine 4,5-Bis(p-Methoxyphenyl)imidazolderivate.

Das Blatt Acta Chim. Budapest 1969, 61 (1), Seiten 69-77 offenbart in seinem Abstract substituierte Benzimidazole., jedoch keine Arylimidazo[1,2-a]thiolanderivate.

Das Blatt Chemical Abstract, Band 78, Nr. 72002k vom 19.3.1973 offenbart phenylsubstituiere 4,5-Diphenylimidazolderivate, auch ringförmige und 4,5-Bis(p-Methoxyphenyl)imidazolderivate; jedoch scheint keine Arylimidazo[1,2-a]thiolanderivate.

Die Schrift DE 2823197 offenbart Imidazolderivate, aber keine arylalkyl-schwefelsubstituierten Imidazolderivate.

Die Schrift WO 91/10662 offenbart zwar lmidazolderivate, die in 2-Stellung linear substituiert sind, aber nicht Arylimidazo[1,2-a]thiolanderivate.

Die Schrift EP372445 offenbart Imidazolcarbamate und -Harnstoffe.

Die Schrift DE 19842833 offenbart 5-Heteroarylimidazolderivate.

Die Schrift WO 95/00501 offenbart verschiedene heterocyclische Cyclooxygenasehemmer, aber keine Arylimidazo[1,2-a]thiolanderivate.

EP-A-0 231 622, US-A-4 064 260 und BENDER P. E. et al. J of Medicinal Chemistry, Bd. 28, Nr. 9, 1985, Seiten 1169-1177 offenbaren eine antiinflammatorische Wirkung von Diar substituierten Imidazothiazolen einschließlich des 4, 5-bis(4-Methoxyphenyl)-imidazo[1,2-a]thiolan S-oxids und S-dioxids.

Keine dieser Schriften offenbart näheres zum Problem der Phototoxizität derartiger Verbindungen bei der topischen Anwendung. Es hat sich aber gezeigt, dass diese Eigenschaft den Einsatz dieser Verbindungen in topischen Zubereitungen stark limitiert.

Ausgehend hiervon stellte sich die Aufgabe, antientzündlich wirkende topische Arzneizubereitungen sowie kosmetische und/oder dermatologische Zubereitungen zu finden, die sich bei genügender Wirksamkeit durch eine geringe Phototoxizität auszeichen und die insbesondere lichtstrapazierte Haut langanhaltend pflegen.

Es hat sich für den Fachmann nicht vorhersehbar herausgestellt, daß topische, antientzündlich wirkende Arzneizubereitungen enthaltend Verbindungen der Formel (DKH) 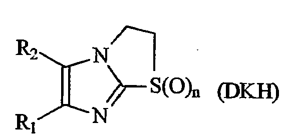 wobei
n die Werte 1 oder 2 hat und
R1 und R2 die Gruppierung 4-Methoxyphenyl darstellen
sowie
topische, antientzündlich wirkende kosmetische und/oder dermatologische Zubereitungen enthaltend Verbindungen der Formel (DKH) wobei
n die Werte 1 oder 2 hat und
R1 und R2 die Gruppierung 4-Methoxyphenyl darstellen,
den Mängeln des Standes der Technik abhelfen.

Diese Zubereitungen weisen gegenüber den Zubereitungen des Standes der Technik folgende Vorteile auf:
die Substanzen können stabil in Formulierungen eingearbeitet werden und weisen nach Auftragen auf die Haut eine gute Penetration an den Wirkort (Epidermis, Dermis, Endothel) auf.

Es war außerdem überraschend dass erfindungsgemäße kosmetische oder dermatologische Formulierungen durch eine geringe Phototoxizität auszeichnen, eine unabdingbare Voraussetzung zum Einsatz der Wirksubstanzen in Kosmetika, die auf lichtexponierte Haut aufgetragen werden, was bekanntlich sehr oft der Fall ist.

Von diesen erfindungsgemäßen Zubereitungen erwartet der Anwender weiterhin, dass sie gut verträglich sind, das gilt insbesondere für Kosmetika mit anti-entzündlichen Wirkstoffen wie den hier beschriebenen, da sie ja bestimmungsgemäß auf sensible Haut, rasur-geschädigter, durch Sonnenbrand gereizte Haut oder anderweitig vorgeschädigte Haut aufgetragen werden. In diesen Fällen ist es also besonders wichtig, dass die Zubereitungen problemlos vertragen werden. Darüber hinaus sollen diese Zubereitungen jedoch trotzdem auch kosmetisch ansprechend sei. All dies erfüllen die erfindungsgemäßen Zubereitungen.

Es wurde weiter gefunden, daß es bevorzugt ist, wenn n =1 ist.

Bevorzugt ist es weiterhin, wenn die Verbindung der Formel (DKH) in Konzentrationen von 10 bis 0,0001 Gew.%, besonders bevorzugt von 0,001 bis 1 Gew.% und ganz besonders bevorzugt von 0,01 bis 0,1 Gew.% vorliegt.

Derartige erfindungsgemäße Zubereitungen werden bevorzugt verwendet, indem die antientzündliche Wirkung gegen Psoriasis, Atopisches Ekzem, Akne, Rosazea, allergische und imtative Kontaktdermatis, Sonnen- und Rasurbrand, Windeldermatitis, seborrhoische Dermatitis, Sonnenallergie (polymorphe Lichtdermatose, Mallorca-Akne) und aktinische Keratosen gerichtet ist. Besonders bevorzugt werden erfindungsgemäße Zubereitungen zur Behandlung und Prophylaxe von Juckreiz, hyperreaktiven Hautzuständen, empfindlicher Haut, photogeschädigter Haut und unreiner Haut eingesetzt.

Eine ganz besonders bevorzugte Verwendung solcher Zubereitungen besteht darin, dass die Zubereitung auf die sonnenexponierte Haut aufgetragen wird.

Die Erfindung umfasst auch die Verwendung von Verbindungen der Formel (DKH) zur Herstellung topischer, antientzündlich wirkender Arzneizubereitungen, wobei n die Werte 1 oder 2 hat und
R1 und R2 die Gruppierung 4-Methoxyphenyl darstellen
sowie die

Verwendung von Verbindungen der Formel (DKH) zur Herstellung topischer, antientzündlich wirkender kosmetischer und/oder dermatologischer Zubereitungen, wobei n die Werte 1 oder 2 hat und
R1 und R2 die Gruppierung 4-Methoxyphenyl darstellen.

All diese Zubereitungen und Verwendungen helfen den Mängeln des Standes der Technik ab, indem sie gegenüber den Zubereitungen des Standes der Technik weitere Vorteile aufweisen.

Die Formulierungen im Sinne der vorliegenden Erfindung sind in jeglicher Hinsicht überaus befriedigende Präparate, die sich durch eine langanhaltende Pflegewirkung auszeichnen. Es war für den Fachmann nicht vorauszusehen gewesen, dass die erfindungsgemäß verwendeten Formulierungen
lichtstrapazierte und rasurgestresste Haut besser pflegen,
die Nachreaktionen der Haut auf die Einwirkung von UV-Strahlung und auf die (mechanische) Reizung durch Rasieren besser vermindern,
die vom Sonnenbaden und der Rasur gereizte Haut besser beruhigen,
leichten Sonnenbrand und den Rasurbrand schneller zum Abklingen bringen,
besser die Hautglättung fördern,
sich durch bessere Pflegewirkung auszeichnen,
bessere sensorische Eigenschaften, wie beispielsweise die Verteilbarkeit auf der Haut oder das Einzugsvermögen in die Haut, aufweisen würden als die Zubereitungen des Standes der Technik.

Es war ferner erstaunlich, dass die Zubereitungen im Sinne der vorliegenden Erfindung die Nachreaktionen der Haut auf die Einwirkung von UV-Strahlung und auf die (mechanische) Reizung durch Rasieren auch dann vermindern, wenn sie bereits vor bzw. während eines Sonnenbades und/oder einer Rasur verwendet (d. h. auf die Haut aufgebracht) werden.

Die Erfindung ist selbstverständlich nicht auf Zubereitungen beschränkt, welche nach dem Sonnenbad und/oder der Rasur angewendet werden, sondern umfasst naturgemäß alle kosmetischen und dermatologischen Anwendungen, bei welchen eine stresslindernde Wirkung gewünscht oder von Vorteil sein könnte. Das oben Gesagte zur positiven Wirkung der erfindungsgemäß verwendeten Formulierungen gilt in gleicher Weise bei lichtstrapazierter Haut wie auch bei rasurstrapazierter Haut.

Für 4,5-Bis(4-Methoxypheny))-imidazo[1,2-a]thio)an-1-oxid sowie 4,5-Bis(4-Methoxyphenyl)-imidazo[1,2-a]thiolan-1-dioxid wurde im Photo-Zytotoxizitätstest überraschend gefunden, daß diese im Vergleich zur nichtoxidierten Vergleichsverbindung 4,5-Bis(4-Methoxyphenyl)-imidazo[1,2-a]thiolan eine erheblich reduzierte Phototoxizität aufweisen. Gleichzeitig wurde gefunden, daß die Produktion von Leukotrien B4 in Granulozyten und von Prostaglandin E2 in Fibroblasten deutlich reduziert wird, die Stoffe also sehr wirksam sind.

Als Hautbefeuchtungsmittel lassen sich vorteilhaft Glycerin, Chitosan, Fucogel, Propylenglycol, Dipropylenglycol, Butylenglycol, Mannitol, Milchsäure, Natriumpyrolidoncarbonsäure, Hyaluronsäure, Salze der angegebenen Säuren sowie Glycin, Harnstoff und Salze von Metallen der ersten und zweiten Hauptgruppe verwenden.

Besonders geeignet sind Glycerin, Milchsäure, Butylenglycol, Harnstoff, Hyaluronsäure.

Der Gehalt an Hautbefeuchtungsmitteln beträgt vorteilhaft 3 Gew.% bis 60 Gew.%, vorzugsweise 4 bis 50 Gew.%, insbesondere 5 bis 40 Gew.%, bezogen auf das Gesamtgewicht der Zubereitungen.

Es ist auch vorteilhaft im Sinne der vorliegenden Erfindung, kosmetische und dermatologische Zubereitungen zu erstellen, deren hauptsächlicher Zweck nicht der Schutz vor Sonnenlicht ist, die aber dennoch einen Gehalt an UV-Schutzsubstanzen enthalten. So werden z. B. in Tagescremes oder Makeup-Produkten gewöhnlich UV-A- bzw. UV-B-Filtersubstanzen eingearbeitet. Auch stellen UV-Schutzsubstanzen, ebenso wie Antioxidantien und, gewünschtenfalls, Konservierungsstoffe, einen wirksamen Schutz der Zubereitungen selbst gegen Verderb dar. Günstig sind ferner kosmetische und dermatologische Zubereitungen, die in der Form eines Sonnenschutzmittels vorliegen.

Dementsprechend enthalten die Zubereitungen im Sinne der vorliegenden Erfindung vorzugsweise mindestens eine UV-A- und/oder UV-B-Filtersubstanz. Die Formulierungen können, obgleich nicht notwendig, gegebenenfalls auch ein oder mehrere organische und/oder anorganische Pigmente als UV-Filtersubstanzen enthalten, welche in der Wasser- und/oder der Ölphase vorliegen können.

Bevorzugte anorganische Pigmente sind Metalloxide und/oder andere in Wasser schwerlösliche oder unlösliche Metallverbindungen, insbesondere Oxide des Titans (TiO2), Zinks (ZnO), Eisens (z. B. Fe203), Zirkoniums (ZrO2), Siliciums (SiO2), Mangans (z. B. MnO), Aluminiums (Al203), Cers (z. B. Ce203), Mischoxide der entsprechenden Metalle sowie Abmischungen aus solchen Oxiden, sowie das Sulfat des Bariums (BaS04).

Die Titandioxid- Pigmente können sowohl in der Kristallmodifikation Rutil als auch Anatas vorliegen und können im Sinne der vorliegenden Erfindung vorteilhaft oberflächlich behandelt ("gecoatet") sein, wobei beispielsweise ein hydrophiler, amphiphiler oder hydrophober Charakter gebildet werden bzw. erhalten bleiben soll. Diese Oberflächenbehandlung kann darin bestehen, daß die Pigmente nach an sich bekannten Verfahren mit einer dünnen hydrophilen und/oder hydrophoben anorganischen und/oder organischen Schicht versehen werden. Die verschiedenen Oberflächenbeschichtung können im Sinne der vorliegenden Erfindung auch Wasser enthalten.

Beschriebene beschichtete und unbeschichtete Titandioxide können im Sinne vorliegender Erfindung auch in Form commerziell erhältlicher öliger oder wäßriger Vordispersionen zur Anwendung kommen. Diesen Vordispersionen können vorteilhaft Dispergierhilfmittel und/oder Solubilisationsvermittler zugesetzt sein.

Die erfindungsgemäßen Titandioxide zeichnen sich durch eine Primärpartikelgröße zwischen 10 nm bis 150 nm aus.

| **Handelsname** | **Coating** | **zusätzliche Bestandteile der Vordispersion** | **Hersteller** |
|---|---|---|---|
| MT-100TV | Aluminiumhydroxid Stearinsäure | - | Tayca Corporation |
| MT-100Z | Aluminiumhydroxid Stearinsäure | - | Tayca Corporation |
| MT-100F | Stearinsäure Eisenoxid | - | Tayca Corporation |
| MT-500SAS | Alumina, Silica Silikon | - | Tayca Corporation |
| MT-100AQ | Silica Aluminiumhydroxid Alginsäure | - | Tayca Corporation |
| Eusolex T-2000 | Alumina Simethicone | - | Merck KgaA |
| Eosolex TS | Alumina, Stearinsäure | - | Merck KgaA |
| Titandioxid P25 | None | - | Degussa |
| Titandioxid T805 (Uvinul TiO₂) | Octyltrimethylsilan | - | Degussa |
| UV-Titan X170 | Alumina Dimethicone | - | Kemira |
| UV-Titan X161 | Alumina, Silica Stearinsäure | - | Kemira |
| Tioveil AQ 10PG | Alumina Silica | Wasser Propylenglycol | Solaveil Uniquema |
| Mirasun TiW 60 | Alumina Silica | Wasser | Rhone-Poulenc |

Im Sinne der vorliegenden Erfindung sind besonders bevorzugte Titandioxide das MT-100 Z und MT-100 TV von Tayca Corporation, Eusolex T-2000 und Eusolex TS von Merck und das Titandioxid T 805 von Degussa.

Zinkoxide können im Sinne der vorliegenden Erfindung auch in Form kommerziell erhältlicher öliger oder wäßriger Vordispersionen zur Anwendung kommen. Erfindungsgemäß geeignete Zinkoxidpartikel und Vordispersionen von Zinkoxidpartikeln zeichnen sich durch eine Primärpartikelgröße von < 300 nm aus und sind unter folgenden Handelsbezeichnungen bei den aufgeführten Firmen erhältlich:

| Handelsname | Coating | Hersteller |
|---|---|---|
| Z- Cote HP1 | 2% Dimethicone | BASF |
| Z- Cote | / | BASF |
| ZnO NDM | 5% Dimethicone | H&R |
| MZ 707M | 7% Dimethicone | M. Tayca Corp. |
| Nanox 500 | / | Elementis |
| ZnO Neutral | / | H&R |

Besonderes bevorzugte Zinkoxide im Sinne der Erfindung sind das Z-Cote HP1 von der Firma BASF und das Zinkoxid NDM von der Firma Haarmann & Reimer.

Die Gesamtmenge an einem oder mehreren anorganischen Pigmenten in der fertigen kosmetischen Zubereitung wird vorteilhaft aus dem Bereich 0,1 Gew.-% bis 25 Gew.-% gewählt, vorzugsweise 0,5 Gew.-% bis 18 Gew.-%.

Vorteilhaftes organisches Pigment im Sinne der vorliegenden Erfindung ist das 2,2'-Methylen-bis-(6-(2H-benzotriazol-2-yl)-4-(1,1,3,3-tetramethylbutyl)-phenol) [INCI: Bisoctyltriazol], welches durch die chemische Strukturformel 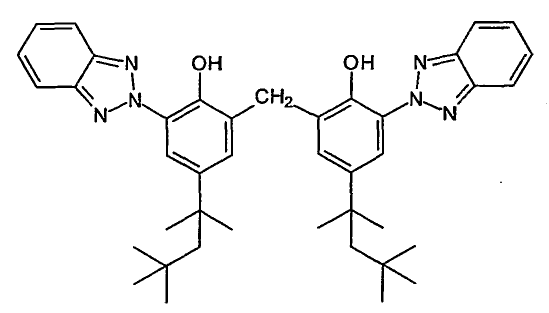 gekennzeichnet ist und unter der Handelsbezeichnung Tinosorb® M bei der CIBA-Chemikalien GmbH erhältlich ist.

Vorteilhafte UV-A-Filtersubstanzen im Sinne der vorliegenden Erfindung sind Dibenzoylmethanderivate, insbesondere das 4-(tert.-Butyl)-4'-methoxydibenzoylmethan (CAS-Nr. 70356-09-1), welches von Givaudan unter der Marke Parsol® 1789 und von Merck unter der Handelsbezeichnung Eusolex® 9020 verkauft wird.

Weitere vorteilhafte UV-A-Filtersubstanzen sind die Phenylen-1,4-bis-(2-benzimidazyl)-3,3'-5,5'-tetrasulfonsäure 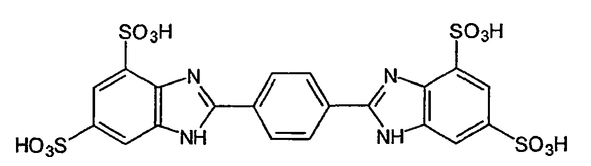 und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz 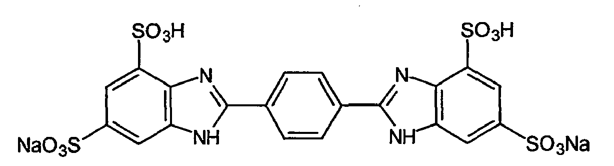 mit der INCI-Bezeichnung Bisimidazylate, welches beispielsweise unter der Handelsbezeichnung Neo Heliopan AP bei Haarmann & Reimer erhältlich ist.

Ferner vorteilhaft sind das 1,4-di(2-oxo-10-Sulfo-3-bornylidenmethyl)-Benzol und dessen Salze (besonders die entprechenden 10-Sutfato-verbindungen, insbesondere das entsprechende Natrium-, Kalium- oder Triethanolammonium-Salz), das auch als Benzol-1,4-di(2-oxo-3-bomylidenmethyl-10-sulfonsäure) bezeichnet wird und sich durch die folgende Struktur auszeichnet:

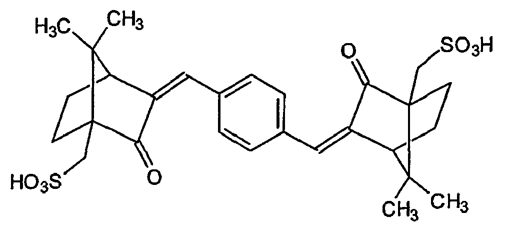

Weitere vorteilhafte UV-A-Filtersubstanzen sind Hydroxybenzophenone, die sich durch die folgende Strukturformel auszeichnen: 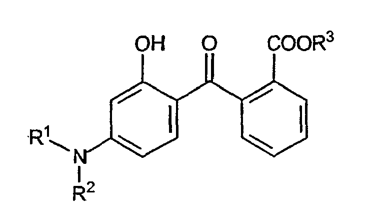 worin
- R¹ und R² unabhängig voneinander Wasserstoff, C₁-C₂₀-Alkyl, C₃-C₁₀-Cycloalkyl oder C₃-C₁₀-Cycloalkenyl bedeuten, wobei die Substituenten R¹ und R² gemeinsam mit dem Stickstoffatom, an das sie gebunden sind, einen 5- oder 6-Ring bilden können und
- R³ einen C₁-C₂₀-Alkyl Rest bedeutet.

Ein besonders vorteilhaftes Hydroxybenzophenon im Sinne der vorliegenden Erfindung ist der 2-(4'-Diethylamino-2'-hydoxybenzoyl)-benzoesäurehexytester (auch: Aminobenzophenon), welcher sich durch folgende Struktur auszeichnet: 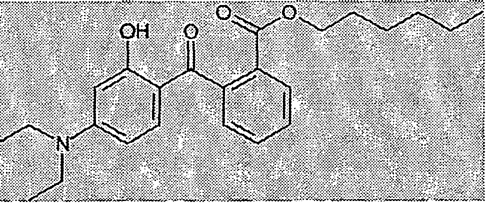 und unter dem Handelsnamen Uvinul A Plus bei der Fa. BASF erhältlich ist.

Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.

Vorteilhafte Breitbandfilter oder UV-B-Filtersubstanzen sind beispielsweise Bis-Resorcinyltriazinderivate mit der folgenden Struktur: 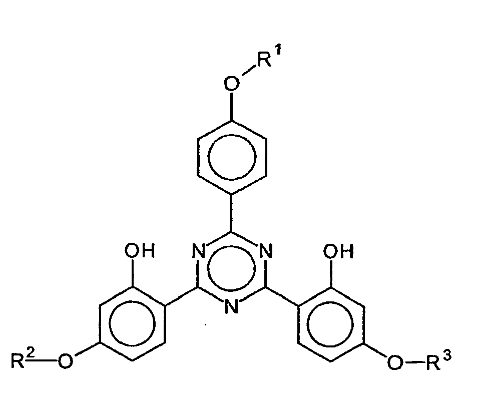 wobei R¹, R² und R³ unabhängig voneinander gewählt werden aus der Gruppe der verzweigten und unverzweigten Alkylgruppen mit 1 bis 10 Kohlenstoffatomen bzw. ein einzelnes Wasserstoffatom darstellen. Insbesondere bevorzugt sind das 2,4-Bis-{[4-(2-Ethyl-hexytoxy}-2-hydroxy]-phenyl}-6-(4-methoxyphenyl)-1,3,5-triazin (INCI: Aniso Triazin), welches unter der Handelsbezeichnung Tinosorb® S bei der CIBA-Chemikalien GmbH erhältlich ist.

Besonders vorteilhafte Zubereitungen im Sinne der vorliegenden Erfindung, die sich durch einen hohen bzw. sehr hohen UV-A-Schutz auszeichnen, enthalten bevorzugt mehrere UV-A- und/oder Breitbandfilter, insbesondere Dibenzoylmethanderivate [beispielsweise das 4-(tert.-Butyl)-4'-methoxydibenzoylmethan], Benzotriazolderivate [beispielsweise das 2,2'-Methylen-bis-(6-(2H-benzotriazol-2-yl)-4-(1,1,3,3-tetramethylbutyl)-phenol)], Phenylen-1,4-bis-(2-benzimidazyl)-3,3'-5,5'-tetrasulfonsäure und/oder ihre Salze, das 1,4-di(2-oxo-10-Sulfo-3-bomylidenmethyl)-Benzol und/oder dessen Salze und/oder das 2,4-Bis-{[4-(2-Ethyl-hexyloxy)-2-hydroxy]-phenyl}-6-(4-methoxyphenyl)-1,3,5-triazin, jeweils einzeln oder in beliebigen Kombinationen miteinander.

Auch andere UV-Filtersubstanzen, welche das Strukturmotiv 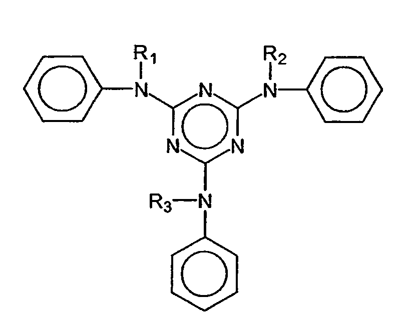 aufweisen, sind vorteilhafte UV-Filtersubstanzen im Sinne der vorliegenden Erfindung, beispielsweise die in der Europäischen Offenlegungsschrift EP 570 838 A1 beschriebenen s-Triazinderivate, deren chemische Struktur durch die generische Formel 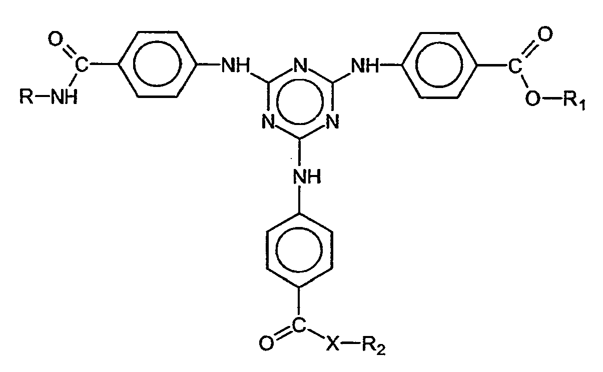 wiedergegeben wird, wobei
- R: einen verzweigten oder unverzweigten C₁-C₁₈-Alkylrest, einen C₅-C₁₂-Cycloalkylrest, gegebenenfalls substituiert mit einer oder mehreren C₁-C₄- Alkylgruppen, darstellt,
- X: ein Sauerstoffatom oder eine NH-Gruppe darstellt,
- R₁: einen verzweigten oder unverzweigten C₁-C₁₈-Alkylrest, einen C₅-C₁₂-Cycloalkylrest, gegebenenfalls substituiert mit einer oder mehreren C₁-C₄- Alkylgruppen, oder ein Wasserstoffatom, ein Alkalimetallatom, eine Ammoniumgruppe oder eine Gruppe der Formel 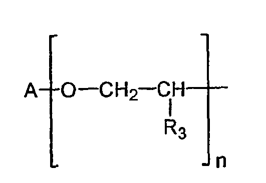 bedeutet, in welcher
A einen verzweigten oder unverzweigten C₁-C₁₈-Alkylrest, einen C₅-C₁₂-Cycloalkyl- oder Arylrest darstellt, gegebenenfalls substituiert mit einer oder mehreren C₁-C₄-Alkylgruppen,
R₃ ein Wasserstoffatom oder eine Methylgruppe darstellt,
n eine Zahl von 1 bis 10 darstellt,
- R₂: einen verzweigten oder unverzweigten C₁-C₁₈-Alkylrest, einen C₅-C₁₂-Cycloalkylrest, gegebenenfalls substituiert mit einer oder mehreren C₁-C₄- Alkylgruppen, darstellt, wenn X die NH-Gruppe darstellt, und
einen verzweigten oder unverzweigten C₁-C₁₈-Alkylrest, einen C₅-C₁₂-Cycloalkylrest, gegebenenfalls substituiert mit einer oder mehreren C₁-C₄- Alkylgruppen, oder ein Wasserstoffatom, ein Alkalimetallatom, eine Ammoniumgruppe oder eine Gruppe der Formel 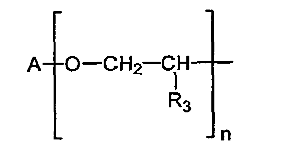 bedeutet, in welcher
A einen verzweigten oder unverzweigten C₁-C₁₈-Alkylrest, einen C₅-C₁₂-Cycloalkyl- oder Arylrest darstellt, gegebenenfalls substituiert mit einer oder mehreren C₁-C₄-Alkylgruppen,
R₃ ein Wasserstoffatom oder eine Methylgruppe darstellt,
n eine Zahl von 1 bis 10 darstellt,
wenn X ein Sauerstoffatom darstellt.

Besonders bevorzugte UV-Filtersubstanz im Sinne der vorliegenden Erfindung ist ferner ein unsymmetrisch substituiertes s-Triazin, dessen chemische Struktur durch die Formel 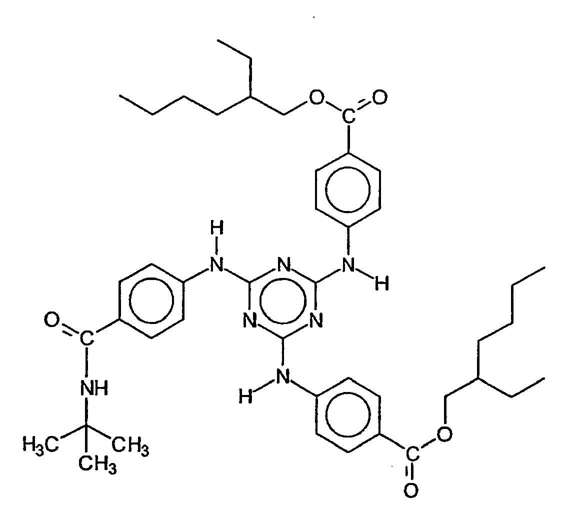 wiedergegeben wird, welches im Folgenden auch als Dioctylbutylamidotriazon (INCl: Dioctylbutamidotriazone) bezeichnet wird und unter der Handelsbezeichnung UVASORB HEB bei Sigma 3V erhältlich ist.

Vorteilhaft im Sinne der vorliegenden Erfindung ist auch ein symmetrisch substituiertes s-Triazin, das 4,4',4"-(1,3,5-Triazin-2,4,6-triyltrümino)-tris-benzoesäure-tris(2-ethylhexylester), synonym: 2,4,6-Tris-[anilino-(p-carbo-2'-ethyl-1'-hexyloxy)]-1,3,5-triazin (INCl: Octyl Triazone), welches von der BASF Aktiengesellschaft unter der Warenbezeichnung UVINUL® T 150 vertrieben wird.

Auch in der Europäischen Offenlegungsschrift 775 698 werden bevorzugt einzusetzende Bis-Resorcinyltriazinderivate beschrieben, deren chemische Struktur durch die generische Formel 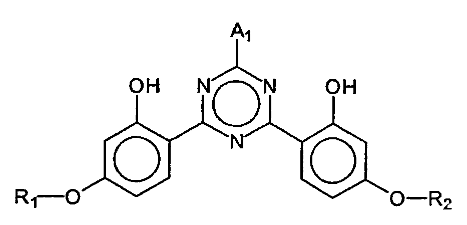 wiedergegeben wird, wobei R₁, R₂ und A₁ verschiedenste organische Reste repräsentieren.

Vorteilhaft im Sinne der vorliegenden Erfindung sind ferner das 2,4-Bis-{[4-(3-sulfonato)-2-hydroxy-propyloxy)-2-hydroxy)-phenyl}-6-(4-methoxyphenyl)-1,3,5-triazin Natriumsalz, das 2,4-Bis-{[4-(3-(2-Propyloxy)-2-hydroxy-propyloxy)-2-hydroxy]-phenyl}-6-(4-methoxyphenyl}-1,3,5-triazin, das 2,4-Bis-{[4-(2-ethyl-hexyloxy)-2-hydroxy]-phenyl}-6-[4-(2-methoxyethyl-carboxyl)-phenylamino]-1,3,5-triazin, das 2,4-Bis-{[4-(3-(2-propyloxy)-2-hydroxy-propyloxy)-2-hydroxy]-phenyl}-6-[4-(2-ethyl-carboxyl)-phenylamino]-1,3,5-triazin, das 2,4-Bis-{[4-(2-ethyl-hexyloxy)-2-hydroxy]-phenyl}-6-(1-methyl-pyrrol-2-yl)-1.3.5-triazin, das 2,4-Bis-{[4-tris(trimethyisi)oxysilylpropyloxy)-2-hydroxy]-phenyl}-6-(4-methoxyphenyl)-1,3,5-triazin, das 2,4-Bis-{[4-(2"-methylpropenyloxy)-2-hydroxy]-phenyl}6-(4-methoxyphenyl)-1,3,5-triazin und das 2,4-Bis-{[4-(1',1',1',3',5',5',5'-Heptamethylsiloxy-2"-methyl-propyloxy)-2-hydroxy]-phenyl}-6-(4-methoxyphenyl)-1,3,5-triazin.

Ein vorteilhafter Breitbandfilter im Sinne der vorliegenden Erfindung ist das 2,2'-Methylen-bis-(6-(2H-benzotriazol-2-yl)-4-(1,1,3,3-tetramethylbutyl)-phenol), welches durch die chemische Strukturformel 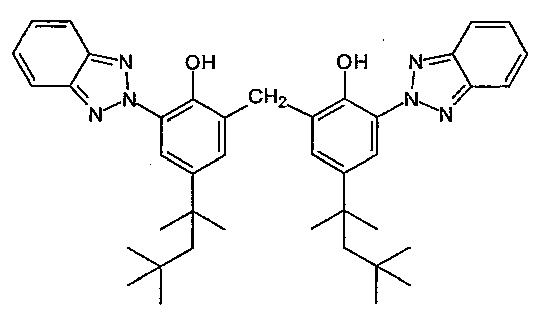 gekennzeichnet ist und unter der Handelsbezeichnung Tinosorb® M bei der CIBA-Chemikalien GmbH erhältlich ist.

Vorteilhafter Breitbandfilter im Sinne der vorliegenden Erfindung ist ferner das 2-(2H-benzotriazol-2-yl)-4-methyl-6-[2-methyl-3-[1,3,3,3-tetramethyl-1-[(trimethylsilyl)oxy]disiloxanyl]propyl]-phenol (CAS-Nr.: 155633-54-8) mit der INCl-Bezeichnung Drometrizole Trisiloxane, welches durch die chemische Strukturformel 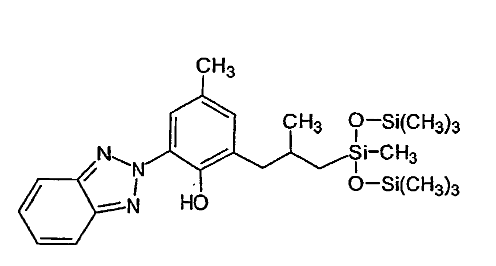 gekennzeichnet ist.

Die UV-B- und/oder Breitband-Filter können öllöslich oder wasserlöslich sein. Vorteilhafte öllösliche UV-B- und/oder Breitband-Filtersubstanzen sind z. B.:
■ 3-Benzylidencampher-Derivate, vorzugsweise 3-(4-Methylbenzyliden)campher, 3-Benzylidencampher;
■ 4-Aminobenzoesäure-Derivate, vorzugsweise 4-(Dimethylamino)-benzoesäure(2-ethylhexyl)ester, 4-(Dimethylamino)benzoesäureamylester;
■ 2,4,6-Trianilino-(p-carbo-2'-ethyl-1'-hexyloxy)-1,3,5-triazin;
■ Ester der Benzalmalonsäure, vorzugsweise 4-Methoxybenzalmalonsäuredi(2-ethylhexyl)-ester,
■ Ester der Zimtsäure, vorzugsweise 4-Methoxyzimtsäure(2-ethylhexyl)ester, 4-Methoxyzimtsäureisopentylester,
■ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon
■ sowie an Polymere gebundene UV-Filter.

Vorteilhafte wasserlösliche UV-B- und/oder Breitband-Filtersubstanzen sind z. B.:
■ Salze der 2-Phenylbenzimidazol-5-sulfonsäure, wie ihr Natrium-, Kalium- oder ihr Triethanolammonium-Salz, sowie die Sulfonsäure selbst;
■ Sulfonsäure-Derivate des 3-Benzylidencamphers, wie z. B. 4-(2-Oxo-3-bomylidenmethyl)-benzolsulfonsäure, 2-Methyl-5-(2-oxo-3-bornylidenmethyl)sulfonsäure und deren Salze.

Besonders vorteilhafte bei Raumtemperatur flüssige UV-Filtersubstanzen im Sinne der vorliegenden Erfindung sind Homomenthylsalicylat (INCl: Homosalate), 2-Ethylhexyl-2-hydroxybenzoat (2-Ethylhexylsalicylat, Octylsalicylat, INCl: Octyl Salicylate), 4-Isopropylbenzylsalicylat und Ester der Zimtsäure, vorzugsweise 4-Methoxyzimtsäure(2-ethylhexyl)ester (2-Ethylhexyl-4-methoxycinnamat, INCI: Octyl Methoxycinnamate) und 4-Methoxyzimtsäureisopentylester (lsopentyl-4-methoxycinnamat, INCI: Isoamyl p-Methoxycinnamate), 3-(4-(2,2-bis Ethoxycarbonylvinyl)-phenoxy)propenyl)-methoxysiloxan/ Dimethylsiloxan - Copolymer (INCI: Dimethicodiethylbenzalmalonat) welches beispielsweise unter der Handelsbezeichnung Parsol® SLX bei Hoffmann La Roche erhältlich ist.

Eine weiterere erfindungsgemäß vorteilhaft zu verwendende Lichtschutzfiltersubstanz ist das Ethylhexyl-2-cyano-3,3-diphenylacrylat (Octocrylen), welches von BASF unter der Bezeichnung Uvinul® N 539 erhältlich ist und sich durch folgende Struktur auszeichnet:

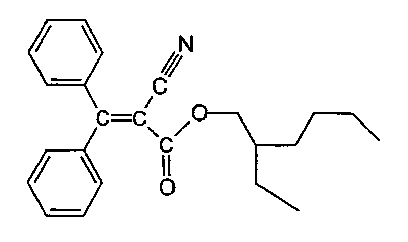

Es kann auch von erheblichem Vorteil sein, polymergebundene oder polymere UV-Filtersubstanzen in Zubereitungen gemäß der vorliegenden Erfindung zu verwenden, insbesondere solche, wie sie in der WO-A-92/20690 beschrieben werden.

Die Liste der genannten UV-Filter, die im Sinne der vorliegenden Erfindung eingesetzt werden können, soll selbstverständlich nicht limitierend sein.

Vorteilhaft enthalten die erfindungsgemäßen Zubereitungen die Substanzen, die UV-Strahlung im UV-A- und/oder UV-B-Bereich absorbieren, in einer Gesamtmenge von z. B. 0,1 Gew.-% bis 30 Gew.-%, vorzugsweise 0,5 bis 20 Gew.-%, insbesondere 1,0 bis 15,0 Gew.-%, jeweils bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

Erfindungsgemäße als Emulsionen vorliegenden Zubereitungen enthalten einen oder mehrere Emulgatoren. Diese Emulgatoren können vorteilhaft gewählt werden aus der Gruppe der nichtionischen, anionischen, kationischen oder amphoteren Emulgatoren.

Unter den nichtionischen Emulgatoren befinden sich
a) Partialfettsäureester und Fettsäureester mehrwertiger Alkohole und deren ethoxylierte Derivate (z. B. Glycerylmonostearate, Sorbitanstearate, Glycerytstearylcitrate, Sucrosestearate)
b) ethoxilierte Fettalkohole und Fettsäuren
c) ethoxilierte Fettamine, Fettsäureamide, Fettsäurealkanolamide
d) Alkylphenolpolyglycolether (z.B. Triton X)

Unter den anionischen Emulgatoren befinden sich
a) Seifen (z. B. Natriumstearat)
b) Fettalkoholsulfate
c) Mono-, Di- und Trialkylphosphosäureester und deren Ethoxylate

Unter den kationischen Emulgatoren befinden sich
a) quaternäre Ammoniumverbindungen mit einem langkettigen aliphatischen Rest z.B. Distearyldimonium Chloride

Unter den amphoteren Emulgatoren befinden sich
a) Alkylamininoalkancarbonsäuren
b) Betaine, Sulfobetaine
c) Imidazolinderivate

Weiterhin gibt es natürlich vorkommende Emulgatoren, zu denen Bienenwachs, Wollwachs, Lecithin und Sterole gehören.

O/W-Emulgatoren können beispielsweise vorteilhaft gewählt werden aus der Gruppe der polyethoxylierten bzw. polypropoxylierten bzw. polyethoxylierten und polypropoxylierten Produkte, z.B.:
- der Fettalkoholethoxylate
- der ethoxylierten Wollwachsalkohole,
- der Polyethylenglycolether der allgemeinen Formel R-O-(-CH₂-CH₂-O-)ₙ-R',
- der Fettsäureethoxylate der allgemeinen Formel

   R-COO-(-CH₂-CH₂-O-)ₙ -H,
- der veretherten Fettsäureethoxylate der allgemeinen Formel

   R-COO-(-CH₂-CH₂-O-)ₙ-R',
- der veresterten Fettsäureethoxylate der allgemeinen Formel

   R-COO-(-CH₂-CH₂-O-)ₙ -C(O)-R',
- der Polyethylenglycolglycerinfettsäureester
- der ethoxylierten Sorbitanester
- der Cholesterinethoxylate
- der ethoxylierten Triglyceride
- der Alkylethercarbonsäuren der allgemeinen Formel

   R-O-(-CH₂-CH₂-O-)ₙ-CH₂-COOH nd n eine Zahl von 5 bis 30 darstellen,
- der Polyoxyethylensorbitolfettsäureester,
- der Alkylethersulfate der allgemeinen Formel R-O-(-CH₂-CH₂-O-)ₙ-SO₃-H
- der Fettalkoholpropoxylate der allgemeinen Formel

   R-O-(-CH₂-CH(CH₃)-O-)ₙ-H,
- der Polypropylenglycolether der allgemeinen Formel

   R-O-(-CH₂-CH(CH₃)-O-)ₙ-R'.
- der propoxylierten Wollwachsalkohole,
- der veretherten Fettsäurepropoxylate

   R-COO-(-CH₂-CH(CH₃)-O-)ₙ-R',
- der veresterten Fettsäurepropoxylate der allgemeinen Formel

   R-COO-(-CH₂-CH(CH₃)-O-)ₙ-C(O)-R',
- der Fettsäurepropoxylate der allgemeinen Formel

   R-COO-(-CH₂-CH(CH₃)-O-)ₙ-H,
- der Polypropylenglycolglycerinfettsäureester
- der propoxylierten Sorbitanester
- der Cholesterinpropoxylate
- der propoxylierten Triglyceride
- der Alkylethercarbonsäuren der allgemeinen Formel

   R-O-(-CH₂-CH(CH₃)O-)ₙ-CH₂-COOH
- der Alkylethersulfate bzw. die diesen Sulfaten zugrundeliegenden Säuren der allgemeinen Formel R-O-(-CH₂-CH(CH₃)-O-)ₙ-SO₃-H
- der Fettalkoholethoxylate/propoxylate der allgemeinen Formel

   R-O-Xₙ-Yₘ-H,
- der Polypropylenglycolether der allgemeinen Formel

   R-O-Xₙ-Yₘ-R',
- der veretherten Fettsäurepropoxylate der allgemeinen Formel

   R-COO-Xₙ-Yₘ-R',
- der Fettsäureethoxylate/propoxylate der allgemeinen Formel

   R-COO-Xₙ-Yₘ-H,.

Erfindungsgemäß besonders vorteilhaft werden die eingesetzten polyethoxylierten bzw. polypropoxylierten bzw. polyethoxylierten und polypropoxytierten O/W-Emulgatoren gewählt aus der Gruppe der Substanzen mit HLB-Werten von 11 -18, ganz besonders vorteilhaft mit mit HLB-Werten von 14,5 - 15,5, sofern die O/W-Emulgatoren gesättigte Reste R und R' aufweisen. Weisen die O/W-Emulgatoren ungesättigte Reste R und/oder R' auf, oder liegen Isoalkylderivate vor, so kann der bevorzugte HLB-Wert solcher Emulgatoren auch niedriger oder darüber liegen.

Es ist von Vorteil, die Fettalkoholethoxylate aus der Gruppe der ethoxylierten Stearylalkohole, Cetylalkohole, Cetylstearylalkohole (Cetearylalkohole) zu wählen. Insbesondere bevorzugt sind:

Polyethylenglycol(13)stearylether (Steareth-13), Polyethylenglycol(14)stearylether (Steareth-14), Polyethylenglycol(15)stearylether (Steareth-15), Polyethylenglycol(16)stearylether (Steareth-16), Polyethylenglycol(17)stearylether (Steareth-17), Polyethylenglycol(18)stearylether (Steareth-18), Polyethylenglycol(19)stearylether (Steareth-19), Polyethylenglycol(20)-stearylether (Steareth-20),

Polyethylenglycol(12)isostearylether (lsosteareth-12), Polyethylenglycol(13)isostearylether (lsosteareth-13), Polyethylenglycol(14)isostearylether (lsosteareth-14), Polyethylenglycol-(15)isostearylether (lsosteareth-15), Polyethylenglycol(16)isostearylether (lsosteareth-16), Polyethylenglycol(17)isostearylether (lsosteareth-17), Polyethylenglycol(18)isostearylether (Isosteareth-18), Polyethylenglycol(19)isostearylether (lsosteareth-19), Polyethylenglycol-(20)isostearylether (Isosteareth-20),

Polyethylenglycol(13)cetylether (Ceteth-13), Polyethylenglycol(14)cetylether (Ceteth-14), Polyethylenglycol(15)cetylether (Ceteth-15), Polyethylenglycol(16)cetylether (Ceteth-16), Polyethylenglycol(17)cetylether (Ceteth-17), Polyethylenglycol(18)cetylether (Ceteth-18), Polyethylenglycol(19)cetylether (Ceteth-19), Polyethylenglycol(20)cetylether (Ceteth-20),

Polyethylenglycol(13)isocetylether (lsoceteth-13), Polyethylenglycol(14)isocetylether (Isoceteth-14), Polyethylenglycol(15)isocetylether (Isoceteth-15), Polyethylenglycol(16)-isocetylether (Isoceteth-16), Polyethylenglycol(17)isocetylether (Isoceteth-17), Polyethylenglycol(18)isocetylether (Isoceteth-18), Polyethylenglycol(19)isocetylether (Isoceteth-19), Polyethylenglycol(20)isocetylether (Isoceteth-20),

Polyethylengtycol(12)oleylether (Oleth-12), Polyethylenglycol(13)oleylether (Oleth-13), Polyethylenglycol(14)oleylether (Oleth-14), Polyethylenglycol(15)oleytether (Oleth-15),

Polyethylenglycol(12)laurylether (Laureth-12), Polyethylenglycol(12)isolaurylether (Isolaureth-12).

Polyethylenglycol(13)cetylstearylether (Ceteareth-13), Polyethylenglycol(14)cetylstearylether (Ceteareth-14), Polyethylenglycol(15)cetylstearylether (Ceteareth-15), Polyethylenglycol-(16)cetylstearylether (Ceteareth-16), Polyethylenglycol(17)cetylstearylether (Ceteareth-17), Polyethylenglycol(18)cetylstearylether (Ceteareth-18), Polyethylenglycol(19)cetylstearylether (Ceteareth-19), Polyethylenglycol(20)cetylstearylether (Ceteareth-20),

Es ist ferner von Vorteil, die Fettsäureethoxylate aus folgender Gruppe zu wählen:

Polyethylenglycol(20)stearat, Polyethylenglycol(21)stearat, Polyethylenglycol(22)stearat, Polyethylenglycol(23)stearat, Polyethylenglycof(24)stearat, Polyethylenglycol(25)stearat,

Polyethylenglycol(12)isostearat, Polyethylenglycol(13)isostearat, Polyethylenglycol(14)isostearat, Polyethylenglycol(15)isostearat, Polyethylenglycol(16)isostearat, Polyethylenglycol-(17)isostearat, Polyethylenglycol(18)isostearat, Polyethylenglycol(19)isostearat, Polyethylenglycol(20)isostearat, Polyethylenglycol(21)isostearat, Polyethylenglycol(22)isostearat, Polyethylenglycol(23)isostearat, Polyethylenglycol(24)isostearat, Polyethylenglycol(25)isostearat,

Polyethylenglycol(12)oteat, Polyethylenglycol(13)oleat, Polyethylenglycol(14)oleat, Polyethylenglycol(15)oleat, Polyethylenglycol(16)oleat, Polyethylenglycol(17)oleat, Polyethylenglycol-(18)oleat, Polyethylenglycot(19)oleat, Polyethylenglycol(20)oleat

Als ethoxylierte Alkylethercarbonsäure bzw. deren Salz kann vorteilhaft das Natriumlaureth-11-carboxylat verwendet werden.

Als Alkylethersulfat kann Natrium Laureth 1-4 sulfat vorteilhaft verwendet werden.

Als ethoxyliertes Cholesterinderivat kann vorteilhaft Polyethylenglycol(30)Cholesterylether verwendet werden. Auch Polyethylenglycol(25)Sojasterol hat sich bewährt.

Als ethoxylierte Triglyceride können vorteilhaft die Polyethylenglycol(60) Evening Primrose Glycerides verwendet werden (Evening Primrose = Nachtkerze)

Weiterhin ist von Vorteil, die Polyethylenglycolglycerinfettsäureester aus der Gruppe Polyethylenglycol(20)glyceryllaurat, Polyethylenglycol(21)glyceryllaurat, Polyethylenglycol(22)-glyceryllaurat, Polyethylenglycol(23)glyceryllaurat, Polyethylenglycol(6)glycerylcaprat/caprinat, Polyethylenglycol(20)glyceryloleat, Polyethylenglycol(20)glycerylisostearat, Polyethylenglycol(18)glyceryloleat/cocoat zu wählen.

Es ist ebenfalls günstig, die Sorbitanester aus der Gruppe Polyethylenglycoi(20)sorbitanmonolaurat, Polyethylenglycol(20)sorbitanmonostearat, Polyethylenglycol(20)sorbitanmonoisostearat, Polyethylenglycol(20)sorbitanmonopalmitat, Polyethylenglycol(20)sorbitanmonooleat zu wählen.

Als vorteilhafte W/O-Emulgatoren können eingesetzt werden: Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12 - 18 C-Atomen, Diglycerinester gesättigter und/oder ungesättigter, verzweigter und/oder unverzweigter Alkancarbonsäuren einer Kettenlänge von 8 bis 24, insbesondere 12 - 18 C-Atomen, Monoglycerinether gesättigter und/oder ungesättigter, verzweigter und/oder unverzweigter Alkohole einer Kettenlänge von 8 bis 24, insbesondere 12 -18 C-Atomen, Diglycerinether gesättigter und/oder ungesättigter, verzweigter und/oder unverzweigter Alkohole einer Kettenlänge von 8 bis 24, insbesondere 12 -18 C-Atomen, Propylenglycolester gesättigter und/oder ungesättigter, verzweigter und/oder unverzweigter Alkancarbonsäuren einer Kettenlänge von 8 bis 24, insbesondere 12 - 18 C-Atomen sowie Sorbitanester gesättigter und/oder ungesättigter, verzweigter und/oder unverzweigter Alkancarbonsäuren einer Kettenlänge von 8 bis 24, insbesondere 12 - 18 C-Atomen.

Insbesondere vorteilhafte W/O-Emulgatoren sind Glycerylmonostearat, Glycerylmonoisostearat, Glycerylmonomyristat, Glycerylmonooleat, Diglycerylmonostearat, Diglycerylmonoisostearat, Propylenglycolmonostearat, Propylenglycolmonoisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol,

Arachidylalkohol, Behenylalkohol, lsobehenylalkohol, Selachylalkohol, Chimylalkohol, Polyethylenglycol(2)stearylether (Steareth-2), Glycerylmonolaurat, Glycerylmonocaprinat, Glycerylmonocaprylat.

Die erfindungsgemäßen Emulsionen können Farbstoffe und/oder Farbpigmente enthalten. Die Farbstoffe und -pigmente können aus der entsprechenden Positivliste der Kosmetikverordnung bzw. der EG-Liste kosmetischer Färbemittel ausgewählt werden. In den meisten Fällen sind sie mit den für Lebensmittel zugelassenen Farbstoffen identisch. Vorteilhafte Farbpigmente sind beispielsweise Titandioxid, Glimmer, Eisenoxide (z. B. Fe₂O₃, Fe₃O₄, FeO(OH)) und/oder Zinnoxid. Vorteilhafte Farbstoffe sind beispielsweise Carmin, Berliner Blau, Chromoxidgrün, Ultramarinblau und/oder Manganviolett. Es ist insbesondere vorteilhaft, die Farbstoffe und/oder Farbpigmente aus der folgenden Liste zu wählen. Die Colour Index Nummern (CIN) sind dem *Rowe Colour lndex, 3. Auflage, Society of Dyers and Colourists, Bradford, England, 1971* entnommen.

| Chemische oder sonstige Bezeichnung | CIN | Farbe |
|---|---|---|
| Pigment Green | 10006 | grün |
| Acid Green 1 | 10020 | grün |
| 2,4-Dinitrohydroxynaphthalin-7-sulfosäure | 10316 | gelb |
| Pigment Yellow 1 | 11680 | gelb |
| Pigment Yellow 3 | 11710 | gelb |
| Pigment Orange 1 | 11725 | orange |
| 2,4-Dihydroxyazobenzol | 11920 | orange |
| Solvent Red 3 | 12010 | rot |
| 1-(2'-Chlor-4'-nitro-1'-phenylazo)-2-hydroxynaphthalin | 12085 | rot |
| Pigment Red 3 | 12120 | rot |
| Ceresrot; Sudanrot; Fettrot G | 12150 | rot |
| Pigment Red 112 | 12370 | rot |
| Pigment Red 7 | 12420 | rot |

| Pigment Brown 1 | 12480 | braun |
|---|---|---|
| 4-(2'-Methoxy-5'-sulfosäurediethylamid-1'-phenylazo)-3-hydroxy-5"-chloro-2",4"-dimefhoxy-2-naphthoesäureanilid | 12490 | rot |
| Disperse Yellow 16 | 12700 | gelb |
| 1-(4-Sulfo-1-phenylazo)-4-amino-benzol-5-sulfosäure | 13015 | gelb |
| 2,4-Dihydroxy-azobenzol-4'-sulfosäure | 14270 | orange |
| 2-(2,4-Dimethylphenylazo-5-sulfosäure)-1-hydroxynaphthalin-4-sulfosäure | 14700 | rot |
| 2-(4-Sulfo-1-naphthylazo)-1-naphthol-4-sulfosäure | 14720 | rot |
| 2-(6-Sulfo-2,4-xylylazo)-1-naphthol-5-sulfosäure | 14815 | rot |
| 1-(4'-Sulfophenylazo)-2-hydroxynaphthalin | 15510 | orange |
| 1-(2-Sulfosäure-4-chlor-5-carbonsäure-1-phenylazo)-2-hydroxynaphthalin | 15525 | rot |
| 1-(3-Methyl-phenylazo-4-sulfosäure)-2-hydroxynaphthatin | 15580 | rot |
| 1-(4',(8')-Sulfosäurenaphthylazo)-2-hydroxynaphthalin | 15620 | rot |
| 2-Hydroxy-1,2'-azonaphthalin-1'-sulfosäure | 15630 | rot |
| 3-Hydroxy-4-phenylazo-2-naphthylcarbonsäure | 15800 | rot |
| 1-(2-Sulfo-4-methyl-1-phenylazo)-2-naphthylcarbonsäure | 15850 | rot |
| 1-(2-Sulfo-4-methyl-5-chlor-1-phenylazo)-2-hydroxy-naphthalin-3-carbonsäure | 15865 | rot |
| 1-(2-Sulfo-l-naphthylazo)-2-hydroxynaphthalin-3-carbonsäure | 15880 | rot |
| 1-(3-Sulfo-1-phenylazo)-2-naphthol-6-sulfosäure | 15980 | orange |
| 1-(4-Sulfo-1-phenylazo)-2-naphthol-6-sulfosäure | 15985 | gelb |
| Allura Red | 16035 | rot |
| 1-(4-Sulfo-1-naphthylazo)-2-naphthol-3,6-disulfosäure | 16185 | rot |
| Acid Orange 10 | 16230 | orange |
| 1-(4-Sulfo-1-naphthylazo)-2-naphthol-6,8-disulfosäure | 16255 | rot |
| 1-(4-Sulfo-1-naphthylazo)-2-naphthol-3,6,8-trisulfosäure | 16290 | rot |
| 8-Amino-2 -phenylazo-1 -naphthol-3,6-disulfosäure | 17200 | rot |
| Acid Red 1 | 18050 | rot |
| Acid Red 155 | 18130 | rot |
| Acid Yellow 121 | 18690 | gelb |
| Acid Red 180 | 18736 | rot |
| Acid Yellow 11 | 18820 | gelb |
| Acid Yellow 17 | 18965 | gelb |
| 4-(4-Sulfo-1-phenylazo)-1-(4-sulfophenyl)-5-hydroxy-pyrazolon-3-carbonsäure | 19140 | gelb |
| Pigment Yellow 16 | 20040 | gelb |
| 2,6-(4'-Sulfo-2", 4"-dimethyl)-bis-phenylazo)1,3-dihydroxybenzol | 20170 | orange |
| Acid Black 1 | 20470 | schwarz |
| Pigment Yellow 13 | 21100 | gelb |
| Pigment Yellow 83 | 21108 | gelb |
| Solvent Yellow | 21230 | gelb |
| Acid Red 163 | 24790 | rot |
| Acid Red 73 | 27290 | rot |
| 2-[4'-(4"-Sulfo-1"-phenylazoh)-7'-sulfo-1'-naphthylazo]-1-hydroxy-7-aminonaphthalin-3,6-disulfosäure | 27755 | schwarz |
| 4'-[(4"-Sulfo-1"-phenylazo)-7'-sulfo-1'-naphthylazo]-1-hydroxy-8-acetyl-aminonaphthalin-3,5-disulfosäure | 28440 | schwarz |
| Direct Orange 34, 39, 44, 46, 60 | 40215 | orange |
| Food Yellow | 40800 | orange |
| trans-β-Apo-8'-Carotinaldehyd (C₃₀) | 40820 | orange |
| trans-Apo-8'-Carotinsäure (C₃₀)-ethylester | 40825 | orange |
| Canthaxanthin | 40850 | orange |
| Acid Blue 1 | 42045 | blau |
| 2,4-Disulfo-5-hydroxy-4'-4"-bis-(diethylamino)triphenyl-carbinol | 42051 | blau |
| 4-[(-4-N-Ethyl-p-sulfobenzylamino)-phenyl-(4-hydroxy-2-sulfophenyl)-(methylen)-1-(N-ethylN-p-sulfobenzyl)-2,5-cyclohexadienimin] | 42053 | grün |
| Acid Blue 7 | 42080 | blau |
| (N-Ethyl-p-sulfobenzyl-amino)-phenyl-(2-sulfophenyl)-methylen-(N-ethyl-N-p-sulfo-benzyl)Δ^{2,5}-cyclohexadienimin | 42090 | blau |
| Acid Green 9 | 42100 | grün |
| Diethyl-di-sulfobenzyl-di-4-amino-2-chlor-di-2-methyl-fuchsonimmonium | 42170 | grün |
| Basic Violet 14 | 42510 | violett |
| Basic Violet 2 | 42520 | violett |
| 2'-Methyl-4'-(N-ethyl-N-m-sulfobenzyl)-amino-4"-(N-diethyl)-amino-2-methyl-N-ethylN-m-sulfobenzyl-fuchsonimmonium | 42735 | blau |
| 4'-(N-Dimethyl)-amino-4"-(N-phenyl)-aminonaphtho-N-dimethylfuchsonimmonium | 44045 | blau |
| 2-Hydroxy-3,6-disulfo-4,4'-bis-dimethylaminonaphthofuchsonimmonium | 44090 | grün |
| Acid Red 52 | 45100 | rot |
| 3-(2'-Methylphenylamino)-6-(2'-methyl-4'-sulfophenylamino)-9-(2"-carboxyphenyl)-xantheniumsalz | 45190 | violett |
| Acid Red 50 | 45220 | rot |
| Phenyl-2-oxyfluoron-2-carbonsäure | 45350 | gelb |
| 4,5-Dibromfluorescein | 45370 | orange |
| 2,4,5,7-Tetrabromfluorescein | 45380 | rot |
| Solvent Dye | 45396 | orange |
| Acid Red 98 | 45405 | rot |
| 3',4',5',6'-Tetrachlor-2,4,5,7-tetrabromfluorescein | 45410 | rot |
| 4,5-Diiodfluorescein | 45425 | rot |
| 2,4,5,7-Tetraiodfluorescein | 45430 | rot |
| Chinophthalon | 47000 | gelb |
| Chinophthalon-disulfosäure | 47005 | gelb |
| Acid Violet 50 | 50325 | violett |
| Acid Black 2 | 50420 | schwarz |
| Pigment Violet 23 | 51319 | violett |
| 1,2-Dioxyanthrachinon, Calcium-Aluminiumkomplex | 58000 | rot |
| 3-Oxypyren-5,8,10-sulfosäure | 59040 | grün |
| 1-Hydroxy-4-N-phenyl-aminoanthrachinon | 60724 | violett |
| 1-Hydroxy-4-(4'-methylphenylamino)-anthrachinon | 60725 | violett |
| Acid Violet 23 | 60730 | violett |
| 1,4-Di(4'-methyl-phenylamino)-anthrachinon | 61565 | grün |
| 1,4-Bis-(o-sulfo-p-toluidino)-anthrachinon | 61570 | grün |
| Acid Blue 80 | 61585 | blau |
| Acid Blue 62 | 62045 | blau |
| N,N'-Dihydro-1,2,1',2'-anthrachinonazin | 69800 | blau |
| Vat Blue 6; Pigment Blue 64 | 69825 | blau |
| Vat Orange 7 | 71105 | orange |
| Indigo | 73000 | blau |
| Indigo-disulfosäure | 73015 | blau |
| 4,4'-Dimethyl-6,6'-dichlorthioindigo | 73360 | rot |
| 5,5'-Dichlor-7,7'-dimethylthioindigo | 73385 | violett |
| Quinacridone Violet 19 | 73900 | violett |
| Pigment Red 122 | 73915 | rot |
| Pigment Blue 16 | 74100 | blau |
| Phthalocyanine | 74160 | blau |
| Direct Blue 86 | 74180 | blau |
| Chlorierte Phthalocyanine | 74260 | grün |
| Natural Yellow 6,19; Natural Red 1 | 75100 | gelb |
| Bixin, Nor-Bixin | 75120 | orange |
| Lycopin | 75125 | gelb |
| trans-alpha-, beta- bzw. gamma-Carotin | 75130 | orange |
| Keto- und/oder Hydroxylderivate des Carotins | 75135 | gelb |
| Guanin oder Perlglanzmittel | 75170 | weiß |
| 1,7-Bis-(4-hydroxy-3-methoxyphenyl)1,6-heptadien-3,5-dion | 75300 | gelb |
| Komplexsalz (Na, Al, Ca) der Karminsäure | 75470 | rot |
| Chlorophyll a und b; Kupferverbindungen der Chlorophylle und Chlorophylline | 75810 | grün |
| Aluminium | 77000 | weiß |
| Tonerdehydrat | 77002 | weiß |
| Wasserhaltige Aluminiumsilikate | 77004 | weiß |
| Ultramarin | 77007 | blau |
| Pigment Red 101 und 102 | 77015 | rot |
| Bariumsulfat | 77120 | weiß |
| Bismutoxychlorid und seine Gemische mit Glimmer | 77163 | weiß |
| Calciumcarbonat | 77220 | weiß |
| Calciumsulfat | 77231 | weiß |
| Kohlenstoff | 77266 | schwarz |
| Pigment Black 9 | 77267 | schwarz |
| Carbo medicinalis vegetabilis | 77268:1 | schwarz |
| Chromoxid | 77288 | grün |
| Chromoxid, wasserhaltig | 77289 | grün |
| Pigment Blue 28, Pigment Green 14 | 77346 | grün |
| Pigment Metal 2 | 77400 | braun |
| Gold | 77480 | braun |
| Eisenoxide und -hydoxide | 77489 | orange |
| Eisenoxid | 77491 | rot |
| Eisenoxidhydrat | 77492 | gelb |
| Eisenoxid | 77499 | schwarz |
| Mischungen aus Eisen(II)- und Eisen(III)-hexacyanoferrat | 77510 | blau |
| Pigment White 18 | 77713 | weiß |
| Mangananimoniumdiphosphat | 77742 | violett |
| Manganphosphat; Mn₃(PO₄)₂ - 7 H20 | 77745 | rot |
| Silber | 77820 | weiß |
| Titandioxid und seine Gemische mit Glimmer | 77891 | weiß |
| Zinkoxid | 77947 | weiß |
| 6,7-Dimethyl-9-(1'-D-ribityl)-isoalloxazin, Lactoflavin | | gelb |
| Zuckerkulör | | braun |
| Capsanthin, Capsorubin | | orange |
| Betanin | | rot |
| Benzopyryliumsalze, Anthocyane | | rot |
| Aluminium-, Zink-, Magnesium- und Calciumstearat | | weiß |
| Bromthymolblau | | blau |
| Bromkresolgrün | | grün |
| Acid Red 195 | | rot |

Es kann ferner günstig sein, als Farbstoff eine oder mehrer Substanzen aus der folgenden Gruppe zu wählen: 2,4-Dihydroxyazobenzol, 1-(2'-Chlor-4'-nitro-1'-phenylazo)-2-hydroxynaphthalin, Ceresrot, 2-(4-Sulfo-1-naphthylazo)-1-naphthol-4-sulfosäure, Calciumsalz der 2-Hydroxy-1,2'-azonaphthalin-1'-sulfosäure, Calcium- und Bariumsalze der 1-(2-Sulfo-4-methyl-1-phenylazo)-2-naphthylcarbonsäure, Calciumsalz der 1-(2-Sulfo-1-naphthylazo)-2-hydroxynaphthalin-3-carbonsäure, Aluminiumsalz der 1-(4-Sulfo-1-phenylazo)-2-naphthol-6-sulfosäure, Aluminiumsalz der 1-(4-Sulfo-1-naphthylazo)-2-naphthol-3,6-disulfosäure, 1-(4-Sulfo-1-naphthylazo)-2-naphthol-6,8-disulfosäure, Aluminiumsalz der 4-(4-Sulfo-1-phenylazo)-1-(4-sutfophenyl)-5-hydroxy-pyrazolon-3-carbonsäure, Aluminium- und Zirkoniumsalze von 4,5-Dibromfluorescein, Aluminium- und Zirkoniumsalze von 2,4,5,7-Tetrabromfluorescein, 3',4',5',6'-Tetrachlor-2,4,5,7-tetrabromfluorescein und sein Aluminiumsalz, Aluminiumsalz von 2,4,5,7-Tetraiodfluorescein, Aluminiumsalz der Chinophthalon-disulfosäure, Aluminiumsalz der Indigo-disulfosäure, rotes und schwarzes Eisenoxid (CIN: 77 491 (rot) und 77 499 (schwarz)), Eisenoxidhydrat (CIN: 77 492), Manganammoniumdiphosphat und Titandioxid. Ferner vorteilhaft sind öllösliche Naturfarbstoffe, wie z. B. Paprikaextrakte, -Carotin oder Cochenille.

Vorteilhaft im Sinne der vorliegenden Erfindung sind ferner Gelcrèmes mit einem Gehalt an Perlglanzpigmenten. Bevorzugt sind insbesondere die im folgenden aufgelisteten Arten von Perlglanzpigmenten:
Natürliche Perlglanzpigmente, wie z. B.
   ■ "Fischsilber" (Guanin/Hypoxanthin-Mischkristalle aus Fischschuppen) und
   ■ "Perimutt" (vermahlene Muschelschalen)
Monokristalline Perlglanzpigmente wie z. B. Bismuthoxychlorid (BiOCl)
Schicht-Substrat Pigmente: z. B. Glimmer / Metalloxid
Basis für Perlglanzpigmente sind beispielsweise pulverförmige Pigmente oder Ricinusöldispersionen von Bismutoxychlorid und/oder Titandioxid sowie Bismutoxychlorid und/oder Titandioxid auf Glimmer. Insbesondere vorteihaft ist z. B. das unter der CIN 77163 aufgelistete Glanzpigment.

Vorteilhaft sind ferner beispielsweise die folgenden Perlglanzpigmentarten auf Basis von Glimmer/Metalloxid:

| **Gruppe** | **Belegung / Schichtdicke** | **Farbe** |
|---|---|---|
| Silberweiße Perlglanzpigmente | TiO₂: 40 - 60 nm | silber |
| Interferenzpigmente | TiO₂: 60 - 80 nm | gelb |
| | TiO₂: 80 -100 nm | rot |
| | TiO₂: 100 - 140 nm | blau |
| | TiO₂: 120 -160 nm | grün |
| Farbglanzpigmente | Fe₂O₃ | bronze |
| | Fe₂O₃ | kupfer |
| | Fe₂O₃ | rot |
| | Fe₂O₃ | rotviolett |
| | Fe₂O₃ | rotgrün |
| | Fe₂O₃ | schwarz |
| Kombinationspigmente | TiO₂/ Fe₂O₃ | Goldtöne |
| | TiO₂ / Cr₂O₃ | grün |
| | TiO₂ / Berliner Blau | tiefblau |
| | TiO₂ / Carmin | rot |

Besonders bevorzugt sind z.B. die von der Firma Merck unter den Handelsnamen Timiron, Colorona oder Dichrona erhältlichen Perlglanzpigmente.

Die Liste der genannten Perlglanzpigmente soll selbstverständlich nicht limitierend sein. Im Sinne der vorliegenden Erfindung vorteilhafte Perlglanzpigmente sind auf zahlreichen, an sich bekannten Wegen erhältlich. Beispielsweise lassen sich auch andere Substrate außer Glimmer mit weiteren Metalloxiden beschichten, wie z. B. Silica und dergleichen mehr. Vorteilhaft sind z. B. mit TiO₂ und Fe₂O₃ beschichtete SiO₂-Partikel ("Ronaspheren"), die von der Firma Merck vertrieben werden und sich besonders für die optische Reduktion feiner Fältchen eignen.

Es kann darüber hinaus von Vorteil sein, gänzlich auf ein Substrat wie Glimmer zu verzichten. Besonders bevorzugt sind Perlglanzpigmente, welche unter der Verwendung von SiO₂ hergestellt werden. Solche Pigmente, die auch zusätzlich gonichromatische Effekte haben können, sind z. B. unter dem Handelsnamen Sicopearl Fantastico bei der Firma BASF erhältlich.

Weiterhin vorteilhaft können Pigmente der Firma Engelhard / Mearl auf Basis von Calcium Natrium Borosilikat, die mit Titandioxid beschichtet sind, eingesetzt werden. Diese sind unter dem Namen Reflecks erhältlich. Sie weisen durch ihrer Partikelgröße von 40 -180 m z u-sätzlich zu der Farbe einen Glitzereffekt auf.

Besonders vorteilhaft sind ferner auch Effektpigmente, welche unter der Handelsbezeichnung Metasomes Standard / Glitter in verschiedenen Farben (yellow, red, green, blue) von der Firma Flora Tech erhältlich sind. Die Glitterpartikel liegen hierbei in Gemischen mit verschiedenen Hilfs- und Farbstoffen (wie beispielsweise den Farbstoffen mit den Colour Index (Cl) Nummern 19140, 77007, 77289, 77491) vor.

Die Farbstoffe und Pigmente können sowohl einzeln als auch im Gemisch vorliegen sowie gegenseitig miteinander beschichtet sein, wobei durch unterschiedliche Beschichtungsdicken im allgemeinen verschiedene Farbeffekte hervorgerufen werden. Die Gesamtmenge der Farbstoffe und farbgebenden Pigmente wird vorteilhaft aus dem Bereich von z. B. 0,1 Gew.-% bis 30 Gew.-%, vorzugsweise von 0,5 bis 20 Gew.-%, insbesondere von 1,0 bis 15 Gew.-% gewählt, jeweils bezogen auf das Gesamtgewicht der Zubereitungen.

Erfindungsgemäße Zubereitungen können vorteilhaft auch Puder enthalten. Puder sind aus einer oder mehreren Pudergrundlagen zusammengesetzte, mehr oder weniger feinteitige, pulverförmige Zubereitungen, welchen, ihrem Anwendungszweck entsprechend, ein oder mehrere Wirkstoffe, Konservierungsmittel, Parfümöle, Farbstoffe usw. beigemischt sein können.

Das FDA's OTC Miscellaneous Extemal Panel hat für Puder nachfolgende Definition festgelegt: "Eine homogene Dispersion feinzerteilten, relativ trockenen feinteiligen Materials, welches aus einer oder mehreren Substanzen besteht" (FDC Reports [Pink Sheet] 41, Nr. 33, T&G-4 [Aug. 13, 1979]).

Die Zusammensetzung eines Puders hängt weitgehend von den Aufgaben ab, die er zu erfüllen hat. Puder können aber auch Verdünnungsmittel für Medikamente, z. B. Antibiotika, Sulfonamide usw., sein. Flüssige Puder sind meist aus Talkum, Zinkoxid und/oder Titandioxid, Glycerin und Wasser bestehende dickflüssige Zubereitungen (Schüttelmixturen). Kompaktpuder sind durch hohen Druck brikettierte oder durch Zugabe von Calciumsulfat (Gips) zusammengesinterte Pudergrundlagen.

Zusätzlich werden Puder auch in Aerosolform zur Verfügung gestellt und angewandt, nachdem es gelungen ist, Ventile zu entwickeln, die die Möglichkeit einer Verstopfung der Ventilausführungsgänge weitgehend ausschließen.

Das immer zu befürchtende Sedimentieren der eingearbeiteten Puderteilchen kann ebenfalls verhindert werden, wenn man in die Rezeptur geeignete Suspendiermittel bzw. Suspendierhilfen einarbeitet, beispielsweise Alkalimetall-, Ammonium- oder Aminsalze eines Dialkylsulfosuccinats mit Alkylgruppen von 4-12 C-Atomen, z. B. Natriumdioctylsulfosuccinat (typischerweise ca. 0,002-0,015 Gew.-%), oder eine Alkylbenzolsulfonsäure mit Alkylgruppen von 8-14 C-Atomen, z. B. Natriumdodecylbenzolsulfonat.

Erfindungsgemäße Zubereitungen können auch vorteilhaft Verdicker enthalten. Geeignete Verdicker sind:

Homopolymere der Acrylsäure mit einem Molekulargewicht von 2000000 bis 6000000 wie z.B. Handelsprodukt Carbopole. Weiter Verdicker werden vertrieben unter den Namen Carbopol 940, Carbopol EDTA 2001 oder Modarez V 600 PX .

Polymere aus Acrylsäure und Acrylamid (Natriumsalz) mit einem Molekulargewicht von 2000000 bis 6000000 wie z.B. Hostacerin PN 73 oder das unter dem Namen Amigel vertriebene Sclerotium Gum.

Außerdem geeignet sind Copolymere der Acrylsäure oder der Methacrylsäure wie z.B. Carbopol 1342 oder Permulen TRI.

Weitere Verdickertypen sind Polyglycole, Cellulosederivate, insbesondere Hydroxyalkylcellulosen sowie Alginate, Carageenan und anorganische Verdicker wie z.B. natürliche oder synthetische Bentonite.

Vorteilhaft werden die Antioxidantien gewählt aus der Gruppe bestehend aus Aminosäuren (z.B. Glycin, Lysin, Arginin, Cystein, Histidin, Tyrosin, Tryptophan) und deren Derivate (als Salz-, Ester-, Ether-, Zucker-, Nukleotid-, Nukleosid-, Peptid- und Lipid-Verbidung), Imidazole (z.B. Urocaninsäure) und deren Derivate (als Salz-, Ester-, Ether-, Zucker-, Nukleotid-, Nukleosid-, Peptid- und/oder Lipid-Verbidung), Peptide wie D,L-Camosin, D-Carnosin, L-Carnosin, Anserin und deren Derivate (z.B. als Salz-, Ester-, Ether-, Zucker-, Thiol-, Nukleotid-, Nukleosid-, Peptid- und Lipid-Verbidung), Carotinoide, Carotine (z.B. α-Carotin, β-Carotin, ψ-Lycopin, Phytoen,) und deren Derivate (z. B. als Salz-, Ester-, Ether-, Zucker-, Nukleotid-, Nukleosid-, Peptid- und/oder Lipid-Verbidung), Chlorogensäure und deren Derivate (als Salz- , Ester-, Ether-, Zucker-, Thiol-, Nukleotid-, Nukleosid-, Peptid- und/oder Lipid-Verbidung), Aurothioglucose, Propylthiouracil und andere Thiole (z.B. Thioredoxin, Liponsäure, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoteyl-, Cholesteryl- und Glycerylester) sowie deren Salze, Dilaurylthiodipropionat, Distearylthiodipropionat, Thiodipropionsäure und deren Derivate (als Salz-, Ester-, Ether-, Zucker-, Thiol-, Nukleotid-, Nukleosid-, Peptid- und/oder Lipid-Verbidung) sowie Sulfoximinverbindungen (z.B. Homocysteinsulfoximin, Buthioninsulfone, Penta-, Hexa-, Heptathioninsulfoximin) in sehr geringen verträglichen Dosierungen (z.B. pmol bis µmol/kg). Ferner (Metall)-Chelatoren (z.B. Apoferritin, Desferral, Lactoferrin, α-Hydroxyfettsäuren, Palmitinsäure, Phytinsäure) und deren Derivate (als Salz-, Ester-, Ether-, Zucker-, Thiol-, Nukleotid-, Nukleosid-, Peptid- und/oder Lipid-Verbidung), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Furfurylidensorbitol und dessen Derivate, Ubichinon, Ubichinol, Plastochinon und deren Derivate (als Salz-, Ester-, Ether-, Zucker-, Thiol-, Nukleotid-, Nukleosid-, Peptid- und Lipid-Verbidung), Vitamin C und Derivate (z.B. Ascorbylpalmitat, Mg-Ascorbylphosphat, Ascorbylacetat), Tocopherole und Derivate (z.B. Vitamin-E-acetat), sowie Phenolische Verbindungen und Pflanzenextrakte, diese enthaltend, wie z. B. Flavonoide (z. B. Glycosylrutin, Ferulasäure, Kaffeesäure), Furfurylidenglucitol, Butylhydroxytoluol, Butylhydroxyanisol, Nordihydroguajakharzsäure, Nordihydroguajaretsäure, Trihydroxybutyrophenon und deren Derivate (als Salz-, Ester-, Ether-, Zucker-, Nukleotid-, Nukleosid-, Peptid- und Lipid-Verbidung). Harnsäure und deren Derivate, Mannose und deren Derivate (als Salz-, Ester-, Ether-, Zucker-, Thiol-, Nukleotid-, Nukleosid-, Peptid- und Lipid-Verbidung). Zink und dessen Derivate (z.B. ZnO, ZnSO₄), Selen und dessen Derivate (z.B. Selenmethionin, Ebselen), Stilbene und deren Derivate (z.B. Stilbenoxid, Trans-Stilbenoxid) und die erfindungsgemäß geeigneten Derivate (als Salz-, Ester-, Ether-, Zucker-, Thiol-, Nukleotid-, Nukleosid-, Peptid- und/ oder Lipid-Verbidung) dieser genannten Wirkstoffe.

Es ist erfindungsgemäß bevorzugt, den erfindungsgemäß verwendeten Wirkstoffkombinationen bzw. kosmetischen oder dermatologischen Zubereitungen, solche Wirkstoffkombinationen enthaltend Komplexbildner zuzufügen.

Komplexbildner sind an sich bekannte Hilfsstoffe der Kosmetologie bzw. der medizinischen Galenik. Durch die Komplexierung von störenden Metallen wie Mn, Fe, Cu und anderer können beispielsweise unerwünschte chemische Reaktionen in kosmetischen oder dennatologischen Zubereitungen verhindert werden.

Komplexbildner, insbesondere Chelatoren, bilden mit Metallatomen Komplexe, welche bei Vorliegen eines oder mehrerer mehrbasiger Komplexbildner, also Chelatoren, Metallacyclen darstellen. Chelate stellen Verbindungen dar, in denen ein einzelner Ligand mehr als eine Koordinationsstelle an einem Zentralatom besetzt. In diesem Falle werden also normalerweise gestreckte Verbindungen durch Komplexbildung über ein Metall-Atom od. -lon zu Ringen geschlossen. Die Zahl der gebundenen Liganden hängt von der Koordinationszahl des zentralen Metalls ab. Voraussetzung für die Chelatbildung ist, daß die mit dem Metall reagierende Verbindung zwei oder mehr Atomgruppierungen enthält, die als Elektronendonatoren wirken.

Der oder die Komplexbildner können vorteilhaft aus der Gruppe der üblichen Verbindungen gewählt werden, wobei bevorzugt mindestens eine Substanz aus der Gruppe bestehend aus Weinsäure und deren Anionen, Citronensäure und deren Anionen, Aminopolycarbonsäuren und deren Anionen (wie beispielsweise Ethylendiamintetraessigsäure (EDTA) und deren Anionen, Nitrilotriessigsäure (NTA) und deren Anionen, Hydroxyethylendiaminofiessigsäure (HOEDTA) und deren Anionen, Diethylenaminopentaessigsäure (DPTA) und deren Anionen, trans-1,2-Diaminocyclohexantetraessigsäure (CDTA) und deren Anionen).

Der oder die Komplexbildner sind erfindungsgemäß vorteilhaft in kosmetischen oder dermatologischen Zubereitungen bevorzugt zu 0,001 Gew.% bis 10 Gew.%, bevorzugt zu 0,01 Gew.% bis 5 Gew.%, insbesondere bevorzugt zu 0,05 - 2,0 Gew.-%, bezogen auf das Gesamtgewicht der Zubereitungen, enthalten.

-Dimethicon-Copolyol (und) Polyglyceryl-4-Isostearat (und) Hexyllaurat.

Vorteilhaft, wenn auch nicht zwingend, können die erfindungsgemäßen Zubereitungen Konservierungsmittel enthalten.

Vorteilhafte Konservierungsmittel im Sinne der vorliegenden Erfindung sind beispielsweise Formaldehydabspalter (wie z. B. DMDM Hydantoin, welches beispielsweise unter der Handelsbezeichnung Glydant ^{™} von der Fa. Lonza erhältlich ist), lodopropylbutylcarbamate (z. B. die unter den Handelsbezeichnungen Glycacil-L, Glycacil-S von der Fa. Lonza erhältlichen und/oder Dekaben LMB von Jan Dekker), Parabene (d. h. p-Hydroxybenzoesäurealkylester, wie Methyl-, Ethyl-, Propyl- und/oder Butylparaben), Phenoxyethanol, Ethanol, Benzoesäure und dergleichen mehr. Üblicherweise umfaßt das Konservierungssystem erfindungsgemäß femer vorteilhaft auch Konservierungshelfer, wie beispielsweise Octoxyglycerin, Glycine Soja etc. Diese Liste der vorteilhaften Konservierungsmittel soll keineswegs limitierend sein. Vielmehr sind alle für Kosmetika oder Lebensmittel zugelassenden Konservierungsmittel vorteilhaft im Sinne der vorliegenden Erfindung.

Es ist bei all diesem im Einzelfalle möglich, daß die vorgenannten Konzentrationsangaben leicht über- oder unterschritten werden und dennoch erfindungsgemäße Zubereitungen erhalten werden. Dies kommt angesichts der breit streuenden Vielfalt an geeigneten Komponenten derartiger Zubereitungen für den Fachmann nicht unerwartet, so daß er weiß, daß bei solchen Über- oder Unterschreitungen der Boden der vorliegenden Erfindung nicht verlassen wird.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

### Beispiele

### Beispiel 1:

A) Photozytotoxizität von 4,5-Bis(4-Methoxyphenyl)-imidazo[1,2-a]thiolan-1-oxid sowie 4,5-Bis(4-Methoxyphenyl)-imidazo[1,2-a]thiolan-1-dioxid im Vergleich zu 4,5-Bis(4-Methoxyphenyl)-imidazo[1,2-a]thiolan nach Standardprotokoll "3T3 NRU Phototoxicity Assay" der COLlPA-Validierungsringstudie von 1997. 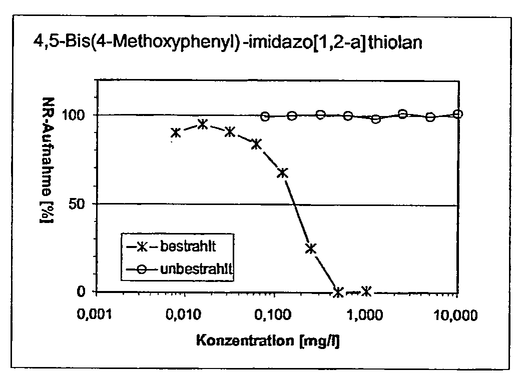
Bei der verwendeten Testmethode wird die Veränderung der Neutralrot-Aufnahme von 3T3-Zellen durch UV-Bestrahlung gemessen. Nur vitale Zellen nehmen den Farbstoff auf, eine verringerte Aufnahme deutet also auf eine Toxizität der zugesetzten Prüfsubstanz hin. Zur Bewertung wird typischerweise der sogenannter NR50-Wert bestimmt, d.h. diejenige Konzentration der Testsubstanz, bei der die Neutralrot-Aufnahme auf 50% des Kontrollwertes abgefallen ist. Um die Phototoxizität zu bestimmen wird dieser Wert für unbestrahlte und bestrahlte Kulturen bestimmt und ins Verhältnis gesetzt. Je geringer der Quotient, desto geringer ist die Phototoxizität (s. Tabelle 1).

| | | | |
|---|---|---|---|
| | NR50 unbestrahlt mg/l | NR50 bestrahlt mg/l | Quotient unbestrahlt/bestrahlt PIF |
| 4,5-Bis(4-Methoxyphenyl)-imidazo[1,2-a]thiolan | > 10 | 0,177 | > 56 |
| 4,5-Bis(4-Methoxyphenyl)-imidazo[1,2-a]thiolan-1-oxid | > 100 | > 42,3 | >2,4 |
| 4,5-Bis(4-Methoxyphenyl)-imidazo[1,2-a]thiolan-1-dioxid | > 50 | 7,77 | > 6,4 |

B) Einfluß von 4,5-Bis(4-Methoxyphenyl)-imidazo[1,2-a]thiolan-1-oxid sowie 4,5-Bis(4-Methoxyphenyl)-imidazo[1,2-a]thiolan-1-dioxid im Vergleich zu 4,5-Bis(4-Methoxyphenyl)-imidazo[1,2-a]thiolan auf die Eicosanoidproduktion.

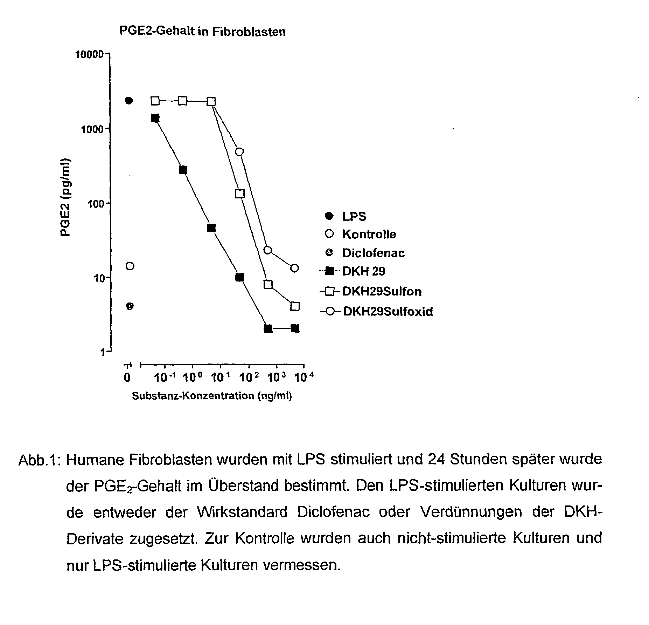

Humane Fibroblasten wurden in Kultur mittels Lipopolysaccharid (LPS) zur Freisetzung von Prostaglandin E₂ (PGE₂) stimuliert. Im Test wurde dann die Inhibition dieser PGE₂ Produktion durch die Imidazole bestimmt. Gute Inhibitoren zeigen eine Wirksamkeit im mikromolaren Bereich und darunter. Abb. 1 zeigt, dass durch die Oxidation des Schwefels zum Sulfoxid bzw. Sulfon die Kapazität zur Inhibition der Cyclooxygenase zwar verringert wird, aber immer noch eine hinreichende Inhibition im Konzentrationsbereich unterhalb eines Mikrogramms pro Milliliter erreicht wird.

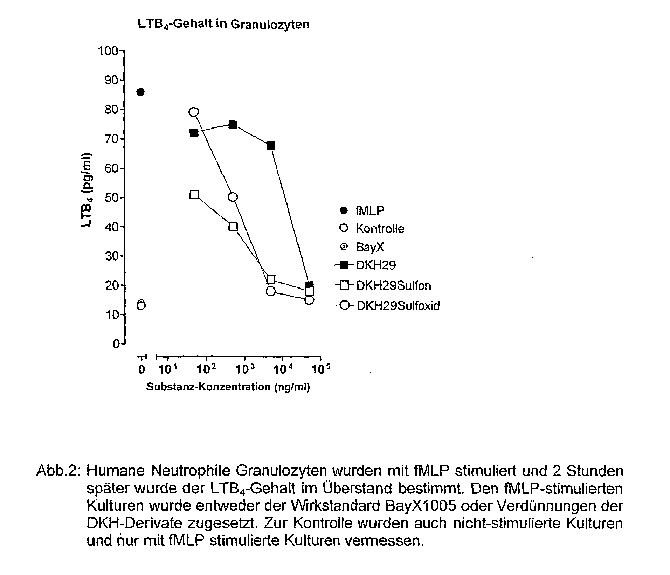

Mittels einer zweiten Testmethode wurde der Einfluß der Imidazole auf die LTB₄-Freisetzung stimulierter Granulozytzen bestimmt. Hierzu wurden humane neutrophile Granulozyten aus dem peripheren Blut isoliert, in Kultur genommen und mit formyliertem Methionyl-Leucyl-Phenylalanin (fMLP) zur LTB4-Produktion stimuliert. Hier zeigte sich, dass sowohl das Sulfoxid als auch das Sulfon eine bessere Inhibition der LTB4-Freisetzung ermöglichen (Abb. 2).

### Rezepturbeispiele

### PIT - Emulsionen

Fett- und Wasserphase wurden getrennt auf 80°C erwärmt. Die Fettphase wird vorgelegt. Bei 80°C wird das Parfüm zugegeben, anschließend wird die Wasserphase zugefügt. Die Emulsion wird unter Rühren auf Raumtemperatur abgekühlt. Eine Homogenisierung ist aufgrund der spontanen Bildung der Emulsion nicht erforderlich.

| | **1** | **2** | **3** | **4** | **5** |
|---|---|---|---|---|---|
| Glycerinmonostearat selbstemulgierend | 0,50 | | 3,00 | 2,00 | 4,00 |
| Polyoxyethylen(12)cetylstearylether | | 5,00 | | 1,00 | 1,50 |
| Polyoxyethylen(20)cetylstearylether | | | | 2,00 | |
| Polyoxyethylen(30)cetylstearylether | 5,00 | | 1,00 | | |
| Stearylalkohol | | | 3,00 | | 0,50 |
| Cetylalkohol | 2,50 | 1,00 | | 1,50 | |
| 2-Ethylhexyl Methoxyzinnamat | | | | 5,00 | 8,00 |
| 2,4-Bis-(4-(2-ethyl-hexyloxy-)2-hydroxyl)-phenyl)-6-(4-methoxyphenyl)-(1,3,5)-triazin | | 1,50 | | 2,00 | 2,50 |
| 1-(4-tert-Butylphenyl)-3-(4-methoxyphenyl)-1,3-propandion | | | 2,00 | | |
| Diethylhexyl Butamidotriazon | 1,00 | 2,00 | | 2,00 | |
| Ethylhexyl Triazon | 4,00 | | 3,00 | 4,00 | |
| 4-Methylbenzyliden Campher | | 4,00 | | | 2,00 |
| Octocrylen | | 4,00 | | | 2,50 |
| Phenylen-1,4-bis-(mononatrium, 2-benzimidazyl-5,7-disulfonsaeure | | | 0,50 | | 1,50 |
| Phenylbenzimidazol Sulfonsäure | 0,50 | | | 3,00 | |
| C12-15 Alkyl Benzoat | | 2,50 | | | 5,00 |
| Titandioxid | 0,50 | 1,00 | | 3,00 | 2,00 |
| Zinkoxid | 2,00 | | 3,00 | 0,50 | 1,00 |
| Dicaprylylether | | | 3,50 | | |
| Butylenglycol-Dicaprylat/-Dicaprat | 5,00 | | | 6,00 | |
| Dicaprylylcarbonat | | | 6,00 | | 2,00 |
| Dimethicon Polydimethylsiloxan | | 0,50 | 1,00 | | |
| Phenylmethylpolysiloxan | 2,00 | | | 0,50 | 0,50 |
| Shea-Butter (Sheabutter) | | 2,00 | | | 0,50 |
| PVP Hexadecencopolymer | 0,50 | | | 0,50 | 1,00 |
| Glycerin | 3,00 | 7,50 | 5,00 | 7,50 | 2,50 |
| Tocopherolacetat | 0,50 | | 0,25 | | 1,00 |
| 4,5-Bis(4-Methoxyphenyl)-imidazo[1,2-a]thiolan-1-oxid | 0,30 | 0,10 | 0,60 | 0,20 | 0,30 |
| Alpha-Glucosylrutin | 0,10 | | 0,20 | | |
| Konservierungsmittel | q.s. | q.s. | q.s. | q.s. | q.s. |
| Ethanol | 3,00 | 2,00 | 1,50 | | 1,00 |
| Parfum | q.s. | q.s. | q.s. | q.s. | q.s. |
| Wasser | ad 100 | ad 100 | ad 100 | ad 100 | ad 100 |

### Beispiele O/W-Creme

| | **1** | **2** | **3** | **4** | **5** |
|---|---|---|---|---|---|
| Glycerylstearatcitrat | | | 2,00 | | 2,00 |
| Glycerylsterat selbstemulgierend | 4,00 | 3,00 | | | |
| PEG-40-Stearat | 1,00 | | | | |
| Polyglyceryl-3-Methylglucose-Distearat | | | | 3,00 | |
| Sorbitanstearat | | | | | 2,00 |
| Stearinsäure | | 1,00 | | | |
| Polyoxyethylen(20)-cetylstearylether | | | | | |
| Stearylalkohol | | | 5,00 | | |
| Cetylalkohol | 3,00 | 2,00 | | 3,00 | |
| Cetylstearylalkohol | | | | | 2,00 |
| C12-15 Alkylbenzoat | | | | | |
| Caprylic-/Capric-Triglycerid | 5,00 | 3,00 | 4,00 | 3,00 | 3,00 |
| Octyldodecanol | | | 2,00 | | 2,00 |
| Dicaprylylether | | 4,00 | | 2,00 | 1,00 |
| Paraffinum liquidum | 5,00 | 2,00 | | 3,00 | |
| Titandioxid | | | 1,00 | | |
| 4-Methylbenzyliden Campher | | | 1,00 | | |
| 1-(4-tert-Butylphenyl)-3-(4-methoxyphenyl)-1,3- | | | 0,50 | | |
| propandion | | | | | |
| 4,5-Bis(4-Methoxyphenyl)-imidazo[1,2-a]thiolan-1-oxid | 0,20 | 0,50 | 0,10 | 1,00 | 0,30 |
| Tocopherol | 0,1 | | | | 0,20 |
| Biotin | | | 0,05 | | |
| Ethylendiamintetraessigsaeure Trinatrium | 0,1 | | 0,10 | 0,1 | |
| Konservierungsmittel | q.s. | q.s. | q.s. | q.s. | q.s. |
| Xanthan Gummi | | | | | |
| Polyacrylsaeure | 3,00 | 0,1 | | 0,1 | 0,1 |
| Natronlauge 45% | q.s | q.s. | q.s. | q.s. | q.s. |
| Glycerin | 5,00 | 3,00 | 4,00 | 3,00 | 3,00 |
| Butylenglycol | | 3,00 | | | |
| Parfum | q.s. | q.s. | q.s. | q.s. | q.s. |
| Wasser | ad 100 | ad 100 | ad 100 | ad 100 | ad 100 |

### Beispiele O/W-Creme

| | **6** | **7** | **8** | **9** | **10** |
|---|---|---|---|---|---|
| Glycerylstearatcitrat | | 2,00 | 2,00 | | |
| Glycerylsterat selbstemulgierend | 5,00 | | | | |
| Stearinsäure | | | | 2,50 | 3,50 |
| Stearylalkohol | 2,00 | | | | |
| Cetylalkohol | | | | 3,00 | 4,50 |
| Cetylstearylalkohol | | 3,00 | 1,00 | | 0,50 |
| C12-15 Alkylbenzoat | | 2,00 | 3,00 | | |
| Caprylic-/Capric-Triglycerid | 2,00 | | | | |
| Octyldodecanol | 2,00 | 2,00 | | 4,00 | 6,00 |
| Dicaprylylether | | | | | |
| Paraffinum liquidum | | 4,00 | 2,00 | | |
| Cyclisches Dimethylpolysiloxan | | | | 0,50 | 2,00 |
| Dimethicon Polydimethylsiloxan | 2,00 | | | | |
| Titandioxid | 2,00 | | | | |
| 4-Methylbenzyliden Campher | 1,00 | | | | 1,00 |
| 1-(4-tert-Butylphenyl)-3-(4-methoxyphenyl)-1,3- | 0,50 | | | | 0,50 |
| propandion | | | | | |
| 4,5-Bis(4-Methoxyphenyl)-imidazo[1,2-a]thiolan-1-dioxid | 0,20 | 0,70 | 0,25 | 1,00 | 0,40 |
| Tocopherol | | | | | 0,05 |
| Ethylendiamintetraessigsaeure Trinatrium | | | 0,20 | | 0,20 |
| Konservierungsmittel | q.s. | q.s. | q.s. | q.s. | q.s. |
| Xanthan Gummi | | | 0,20 | | |
| Polyacrylsaeure | 0,15 | 0,1 | | 0,05 | 0,05 |
| Natronlauge 45% | q.s. | q.s. | q.s. | q.s. | q.s. |
| Glycerin | 3,00 | | 3,00 | 5,00 | 3,00 |
| Butylenglycol | | 3,00 | | | |
| Ethanol | | 3,00 | | 3,00 | |
| Parfum | q.s. | q.s. | q.s. | q.s. | q.s. |
| Wasser | ad 100 | ad 100 | ad 100 | ad 100 | ad 100 |

### Beispiele W/O-Emulsionen

| | **1** | **2** | **3** | **4** | **5** |
|---|---|---|---|---|---|
| Cetyldimethiconcopolyol | | 2,50 | | 4,00 | |
| Polyglyceryl-2-dipolyhydroxystearat | 5,00 | | | | 4,50 |
| PEG-30-dipolyhydroxystearat | | | 5,00 | | |
| 2-Ethylhexyl Methoxyzinnamat | | 8,00 | | 5,00 | 4,00 |
| 2,4-Bis-(4-(2-ethyl-hexyloxy-)2-hydroxyl)-phenyl)-6-(4-methoxyphenyl)-(1,3,5)-triazin | 2,00 | 2,50 | | 2,00 | 2,50 |
| 1-(4-tert-Butylphenyl)-3-(4-methoxyphenyl)-1,3-propandion | | | 2,00 | 1,00 | |
| Diethylhexyl Butamidotriazon | 3,00 | 1,00 | | | 3,00 |
| Ethylhexyl Triazon | | | 3,00 | 4,00 | |
| 4-Methylbenzyliden Campher | | 2,00 | | 4,00 | 2,00 |
| Octocrylen | 7,00 | 2,50 | 4,00 | | 2,50 |
| Diethylhexyl Butamidotriazon | 1,00 | | | 2,00 | |
| Phenylen-1,4-bis-(mononatrium, 2-benzimidazyl-5,7-disulfonsaeure) | 1,00 | 2,00 | 0,50 | | |
| Phenylbenzimidazol Sulfonsäure | 0,50 | | | 3,00 | 2,00 |
| Titandioxid | | 2,00 | 1,50 | | 3,00 |
| Zinkoxid | 3,00 | 1,00 | 2,00 | 0,50 | |
| Paraffinum liquidum | | | 10,0 | | 8,00 |
| C12-15 Alkyl-Benzoat | | | | 9,00 | |
| Dicaprylylether | 10,00 | | | | 7,00 |
| Butylen-Glycol-Dicaprylat/-Dicaprat | | | 2,00 | 8,00 | 4,00 |
| Dicaprylylcarbonat | 5,00 | | 6,00 | | |
| Dimethicon Polydimethylsiloxan | | 4,00 | 1,00 | 5,00 | |
| Phenylmethylpolysiloxan | 2,00 | 25,00 | | | 2,00 |
| Shea Butter | | | 3,00 | | |
| PVP Hexadecencopolymer | 0,50 | | | 0,50 | 1,00 |
| Octoxyglycerin | | 0,30 | 1,00 | | 0,50 |
| Glycerin | 3,00 | 7,50 | | 7,50 | 2,50 |
| Glycinsoja | | 1,00 | 1,50 | | |
| Magnesiumsulfat | 1,00 | 0,50 | | 0,50 | |
| Magnesiumchlorid | | | 1,00 | | 0,70 |
| Tocopherolacetat | 0,50 | | 0,25 | | 1,00 |
| 4,5-Bis(4-Methoxyphenyl)-imidazo[1,2-a]thiolan-1-oxid | 0,10 | 0,60 | 1,00 | 1,00 | 0,80 |
| Konservierungsmittel | q.s. | q.s. | q.s. | q.s. | q.s. |
| Ethanol | 3,00 | | 1,50 | | 1,00 |
| Parfum | q.s. | q.s. | q.s. | q.s. | q.s. |
| Wasser | ad 100 | ad 100 | ad 100 | ad 100 | ad 100 |

### Beispiele W/O Emulsionen

| | **6** | **7** |
|---|---|---|
| Polyglyceryl-2-dipolyhydroxystearat | 4,00 | 5,00 |
| PEG-30-dipolyhydroxystearat | | |
| Lanolinalkohol | 0,50 | 1,50 |
| lsohexadecan | 1,00 | 2,00 |
| Myristyl-Myristat | 0,50 | 1,50 |
| Vaseline | 1,00 | 2,00 |
| 1-(4-tert-Butylphenyl)-3-(4-methoxyphenyl)-1,3-propandion | 0,50 | 1,50 |
| 4-Methylbenzyliden Campher | | 3,00 |
| Butylen-Glycol-Dicaprylat/-Dicaprat | 4,00 | 5,00 |
| Shea Butter | | 0,50 |
| Butylenglycol | | 6,00 |
| Octoxyglycerin | | 3,00 |
| Glycerin | 5,00 | |
| Tocopherolacetat | 0,50 | 1,00 |
| 4,5-Bis(4-Methoxyphenyl)-imidazo[1,2-a]thiolan-1-oxid | 0,20 | 0,25 |
| Trisodium EDTA | 0,20 | 0,20 |
| Konservierungsmittel | q.s. | q.s. |
| Ethanol | | 3,00 |
| Parfum | q.s. | q.s. |
| Wasser | ad 100 | ad 100 |

### Beispiele Hydrodispersionen

| | **1** | **2** | **3** | **4** | **5** |
|---|---|---|---|---|---|
| Polyoxyethylen (20)cetylstearylether | 1,00 | | | 0,5 | |
| Cetylalkohol | | | 1,00 | | |
| Natriumpolyacrylat | | 0,20 | | 0,30 | |
| Acrylate /C10-30-Alkyl-Acrylat Crosspolymer | 0,50 | | 0,40 | 0,10 | 0,10 |
| Xanthan Gummi | | 0,30 | 0,15 | | 0,50 |
| 2-Ethylhexyl Methoxyzinnamat | | | | 5,00 | 8,00 |
| 2,4-Bis-(4-(2-ethyl-hexyloxy-)2-hydroxyl)-phenyl)-6-(4-methoxyphenyl)-(1,3,5)-triazin | | 1,50 | | 2,00 | 2,50 |
| 1-(4-tert-Butylphenyl)-3-(4-methoxyphenyl)-1,3-propandion | 1,00 | | 2,00 | | |
| Diethylhexyl Butamidotriazon | | 2,00 | | 2,00 | 1,00 |
| Ethylhexyl Triazon | 4,00 | | 3,00 | 4,00 | |
| 4-Methylbenzyliden Campher | | 4,00 | | | 2,00 |
| Octocrylen | | 4,00 | 4,00 | | 2,50 |
| Phenylen-1,4-bis-(mononatrium, 2-benzimidazyl-5,7-disulfonsaeure | 1,00 | | 0,50 | | 2,00 |
| Phenylbenzimidazol Sulfonsäure | 0,50 | | | 3,00 | |
| Titandioxid | 0,50 | | 2,00 | 3,00 | 1,00 |
| Zinkoxid | 0,50 | 1,00 | 3,00 | | 2,00 |
| C12-15 Alkyl Benzoat | 2,00 | 2,50 | | | |
| Dicaprylylether | | 4,00 | | | |
| Butylenglycol-Dicaprylat/-Dicaprat | 4,00 | | 2,00 | 6,00 | |
| Dicaprylylcarbonat | | 2,00 | 6,00 | | |
| Dimethicon Polydimethylsiloxan | | 0,50 | 1,00 | | |
| Phenylmethylpolysiloxan | 2,00 | | | 0,50 | 2,00 |
| Shea Butter | | 2,00 | | | |
| PVP Hexadecencopolymer | 0,50 | | | 0,50 | 1,00 |
| Octoxyglycerin | | | 1,00 | | 0,50 |
| Glycerin | 3,00 | 7,50 | | 7,50 | 2,50 |
| Glycinsoja | | | 1,50 | | |
| Tocopherolacetat | 0,50 | | 0,25 | | 1,00 |
| 4,5-Bis(4-Methoxyphenyl)-imidazo[1,2-a]thiolan-1-oxid | 0,15 | 0,60 | 1,00 | 1,00 | 0,80 |
| Konservierungsmittel | q.s. | q.s. | q.s. | q.s. | q.s. |
| Ethanol | 3,00 | 2,00 | 1,50 | | 1,00 |
| Parfum | q.s. | q.s. | q.s. | q.s. | q.s. |
| Wasser | ad 100 | ad 100 | ad 100 | ad 100 | ad 100 |

### Beispiel (Gelcreme):

| | |
|---|---|
| Acrylat /C10-30 Alkylacrylat Crosspolymer | 0,40 |
| Polyacrylsaeure | 0,20 |
| Xanthan Gummi | 0,10 |
| Cetearylalkohol | 3,00 |
| C12-15 Alkylbenzoat | 4,00 |
| Caprylic/Capric Triglycerid | 3,00 |
| Cyclisches Dimethylpolysiloxan | 5,00 |
| Dimeticon Polydimethylsiloxan | 1,00 |
| 4,5-Bis(4-Methoxyphenyl)-imidazo[1,2-a]thiolan-1-oxid | 0,20 |
| Glycerin | 3,00 |
| Natriumhydroxid | q.s. |
| Konservierungsmittel | q.s. |
| Parfum | q.s. |
| Wasser | ad 100 |
| pH-Wert eingestellt auf 6.0 | |

### Beispiel (W/O-Creme)

| | |
|---|---|
| Polyglyceryl-3-Diisostearate | 3,50 |
| Glycerin | 3,00 |
| Polyglyceryl-2-Dipolyhydroxystearate | 3,50 |
| 4,5-Bis(4-Methoxyphenyl)-imidazo[1,2-a]thiolan-1-oxid | 0,50 |
| Konservierungsmittel | q.s. |
| Parfum | q.s. |
| Wasser | ad 100 |
| Magnesiumsulfat | 0,6 |
| Isopropylstearat | 2,0 |
| Caprylylether | 8,0 |
| Cetearylisononanoat | 6,0 |

### Beispiel (W/O/W-Creme):

| | |
|---|---|
| Glycerylstearat | 3,00 |
| PEG-100-Stearat | 0,75 |
| Behenylalkohol | 2,00 |
| Caprylic-/Capric-Triglycerid | 8,0 |
| Octyldodecanol | 5,00 |
| C12-15 Alkylbenzoat | 3,00 |
| 4,5-Bis(4-Methoxyphenyl)-imidazo[1,2-a]thiolan-1-oxid | 1,00 |
| Magnesiumsulfat (MgSO4) | 0,80 |
| Ethylendiamintetraessigsaeure | 0,10 |
| Konservierungsmittel | q.s. |
| Parfum | q.s. |
| Wasser | ad 100 |
| pH-Wert eingestellt auf 6.0 | |

### Beispiel (O/W-Emulsion):

| | |
|---|---|
| Triceteareth-4-Phosphat | 0,70 |
| Glyceryllanolat | 1,50 |
| Cyclisches Dimethylpolysiloxan | 1,00 |
| Butylenglycol | 3,0 |
| Carbomer | 0,45 |
| Natronlauge 45% | 0,30 |
| 4,5-Bis(4-Methoxyphenyl)-imidazo[1,2-a]thiolan-1-oxid | 0,50 |
| Isopropylpalmitat | 0,50 |
| Ethylendiamintetraessigsaeure | 1,00 |
| Konservierungsmittel | q.s. |
| Parfum | q.s. |
| Wasser | ad 100 |

## Patentansprüche

1. Topische, antientzündlich wirkende Arzneizubereitungen enthaltend Verbindungen der Formel (DKH) 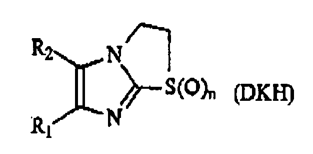 wobei
n die Werte 1 oder 2 hat,
**dadurch gekennzeichnet, dass** R1 und R2 die Gruppierung 4-Methoxyphenyl darstellen.

2. Topische, antlentzündlich wirkende kosmetische und/oder dermatologische Zubereitungen enthaltend Verbindungen der Formel (DKH) wobei
n die Werte 1 oder 2 hat,
**dadurch gekennzeichnet, dass** R1 und R2 die Gruppierung 4-Methoxyphenyl darstellen.

3. Zubereitungen nach mindestens einem der vorangehenden Ansprüche **dadurch gekennzeichnet, dass** n =1 ist.

4. Zubereitungen nach mindestens einem der vorangehenden Ansprüche **dadurch gekennzeichnet, dass** die Verbindung der Formel (DKH) in Konzentrationen von 10 bis 0,0001 Gew.-%, besonders bevorzugt von 0,001 bis 1 Gew.-% und ganz besonders bevorzugt von 0,01 bis 0.1 Gew.-% vorliegt

5. Zubereitungen nach mindestens einem der vorangehenden Ansprüche **dadurch gekennzeichnet, dass** die antientzündliche Wirkung gegen Psoriasis, Atopisches Ekzem, Akne, Rosazea, allergische und irritative Kontaktdermatis, Sonnen- und Rasurbrand, windeldermatitis, seborrhoische Dermatitis, Sonnenallergie (polymorphe Lichtdermatose, Mallorca-Akne) und aktinische Keratosen gerichtet ist.

6. Verwendungen von Zubereitungen nach mindestens einem der vorangehenden Ansprüche zur Herstellung eines Arzneimittels zur Behandlung und Prophylaxe von Juckreiz, hyperreaktiven Hautzuständen, empfindlicher Haut, photogeschädigter Haut und unreiner Haut eingesetzt wird,

7. Verwendung nach Anspruch 6 zur Herstellung eines Arzneimittels zum Auftragen auf die sonnenexponierte Haut.

## Claims

1. Topical, anti-inflammatory medicament preparations comprising compounds of the formula (DKH) 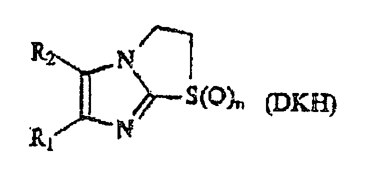 where
n has the values 1 or 2,
**characterized in that** R1 and R2 are the group 4-methoxyphenyl.

2. Topical, anti-inflammatory cosmetic and/or dermatological preparations comprising compounds of the formula (DKH) where
n has the values 1 or 2,
**characterized in that** R1 and R2 are the group 4-methoxyphenyl.

3. Preparations according to at least one of the preceding claims, **characterized in that** n = 1.

4. Preparations according to at least one of the preceding claims, **characterized in that** the compound of the formula (DKH) is present in concentrations of from 10 to 0.0001% by weight, particularly preferably from 0.001 to 1% by weight and very particularly preferably from 0.01 to 0.1% by weight.

5. Preparations according to at least one of the preceding claims, **characterized in that** the anti-inflammatory effect is directed against psoriasis, atopic eczema, acne, rosacea, allergic and irritative contact dermatitis, sunburn and shaving burn, diaper dermatitis, seborrhoeic dermatitis, sun allergy (polymorphous photodermatitis, Mallorca acne) and actinic keratoses.

6. Uses of preparations according to at least one of the preceding claims for producing a medicament for the treatment and prophylaxis of itching, hyperreactive skin conditions, sensitive skin, photodamaged skin and bad skin.

7. Use according to Claim 6 for producing a medicament for application to sun-exposed sun.

## Revendications

1. Préparations médicamenteuses topiques à action anti-inflammatoire, comprenant des composés de formule (DKH) 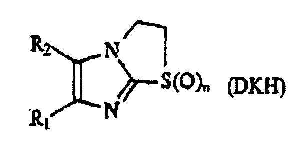 dans laquelle
n vaut 1 ou 2,
**caractérisées en ce que** R1 et R2 représentent le groupement 4-méthoxyphényle.

2. Préparations topiques cosmétiques et/ou dermatologiques à action anti-inflammatoire, comprenant des composés de formule (DKH) dans laquelle
n vaut 1 ou 2,
**caractérisées en ce que** R1 et R2 représentent le groupement 4-méthoxyphényle.

3. Préparations selon au moins l'une des revendications précédentes, **caractérisées en ce que** n vaut 1.

4. Préparations selon au moins l'une des revendications précédentes, **caractérisées en ce que** le composé de formule (DKH) est présent en des concentrations de 10 à 0,0001 % en poids, particulièrement préférablement de 0,001 à 1 % en poids, et tout particulièrement préférablement de 0,01 à 0,1 % en poids.

5. Préparations selon au moins l'une des revendications précédentes, **caractérisées en ce que** l'action anti-inflammatoire est dirigée contre le psoriasis, l'eczéma atopique, l'acné, l'acné rosacée, une dermatite de contact allergique et irritative, un érythème solaire et une brûlure due au rasage, un érythème fessier du nourrisson, la dermatite séborrhéique, une allergie solaire (photodermatite polymorphe, acné de Majorque) et des kératoses actiniques.

6. Utilisation de préparations selon au moins l'une des revendications précédentes, pour la production d'un médicament destiné au traitement et à la prophylaxie d'une démangeaison, d'états cutanés hyper-réactifs, d'une peau sensible, d'une peau abîmée par la lumière et d'une mauvaise peau.

7. Utilisation selon la revendication 6, pour la production d'un médicament destiné à être appliqué sur la peau exposée au soleil.
